(19) Europäisches Patentamt
European Patent Office
Office européen des brevets

(11) Veröffentlichungsnummer: **0 030 920**
**B1**

# (12) EUROPÄISCHE PATENTSCHRIFT

(45) Veröffentlichungstag der Patentschrift: **15.02.84**

(51) Int. Cl.³: **C 07 C 103/52**, A 61 K 37/02

(21) Anmeldenummer: **80810381.6**

(22) Anmeldetag: **08.12.80**

(54) **Acylpeptide und pharmazeutische Präparate davon, sowie deren Herstellung und Verwendung.**

(30) Priorität: **14.12.79 CH 11096/79**

(43) Veröffentlichungstag der Anmeldung: **24.06.81 Patentblatt 81/25**

(45) Bekanntmachung des Hinweises auf die Patenterteilung: **15.02.84 Patentblatt 84/7**

(84) Benannte Vertragsstaaten: **AT BE CH DE FR GB IT LI LU NL SE**

(56) Entgegenhaltungen:
**EP-A-0 011 366**
**DE-A-2 659 758**
**US-A-3 882 098**
**US-A-4 113 714**
**US-A-4 118 380**
**US-A-4 130 554**

(73) Patentinhaber: **CIBA-GEIGY AG, Patentabteilung Postfach, CH-4002 Basel (CH)**

(72) Erfinder: **Rink, Hans, Rebenstrasse 10, CH-4125 Riehen (CH)**
Erfinder: **Sieber, Peter, Bachmattweg 10, CH-4153 Reinach (CH)**
Erfinder: **Kamber, Bruno, Dr., Sonnenweg 9, CH-4144 Arlesheim (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Acylpeptide und pharmazeutische Präparate davon, sowie deren Herstellung und Verwendung

Die Erfindung betrifft Acylpeptide abgeleitet von Somatostatin und seinen Analogen, Verfahren zur Herstellung dieser Acylpeptide, pharmazeutische Präparate enthaltend dieselben, sowie Verwendung dieser Verbindungen bzw. Präparate zu therapeutischen Zwecken. Die erfindungsgemässen Acylpeptide sind Derivate von Somatostatin und von ihm abgeleiteten Analogen, die dadurch gekennzeichnet sind, dass darin die ε-Aminogruppe des Lysinrestes in 9-Stellung, und gegebenenfalls auch die ε-Aminogruppe des Lysinrestes in 4-Stellung und/oder die N-terminale Aminogruppe, durch den Rest einer Carbonsäure substituiert ist; dabei kann die Aminosäuresequenz von Somatostatin durch Auslassen einzelner Aminosäuren oder deren Austausch gegen andere Aminosäuren modifiziert sein.

Somatostatin, ein cyclisches Tetradecapeptid der Formel

H-Ala-Gly-Cys-Lys-Asn-Phe-Phe-Trp-Lys-Thr-Phe-
1 2 3 4 5 6 7 8 9 10 11

Thr-Ser-Cys-OH
12 13 14

[Science *179,* 77 (1973)] hemmt bekanntlich die hypophysengesteuerte Sekretion des Wachstumshormons (Somatotropin). Ausserdem hemmt es die sekretorische Aktivität des endocrinen Pancreas, wie die Sekretion von Insulin und Glucagon. Diese wertvollen Eigenschaften können beim Somatostatin selber nicht zur vollen praktischen Anwendung gelangen, da diese Verbindung eine zu kurze Wirkungsdauer aufweist. Zudem ist es oft bevorzugt, wenn der Wirkstoff seine hemmende Wirkung hauptsächlich auf eines der erwähnten Hormone ausübt. Deshalb wird angestrebt, durch Modifizierung der Grundsequenz, insbesondere durch Auslassen einzelner ursprünglicher Aminosäuren und/oder deren Austausch gegen andere, oft auch «unnatürliche» Aminosäuren, eine Dissociation der Hemmwirkungen sowie eine möglichst lange Wirkungsdauer zu erlangen.

Überraschenderweise wurde nun festgestellt, dass durch eine ganz ungewöhnliche Modifizierung der Grundstruktur, und zwar durch die Acylierung der ε-Aminogruppe des Lysinrestes in der 9-Stellung von Somatostatin oder eines gleichartig wirkenden Strukturanalogen davon mit einer gegebenenfalls substituierten Alkancarbonsäure ein Acylpeptid entsteht, in welchem die ursprüngliche Aktivität der Grundstruktur nicht nur erhalten bleibt, sondern oft im oben diskutierten Sinne, insbesondere in Bezug auf die Wirkungsdauer, weiter gesteigert und vertieft wird. Ein solches Resultat überrascht umsomehr, als dadurch der basische Charakter der endständigen Aminogruppe des $Lys^9$-restes aufgehoben wird, der als unentbehrlich für die biologische Wirkung von Somatostatin und analogen Wirkstoffen gilt.

Die erfindungsgemässen Verbindungen sind oft auch technisch vorteilhaft, da bei ihrer Synthese aus kleineren Bauelementen die Notwendigkeit des selektiven Schutzes der betreffenden Aminogruppen, speziell der ε-Aminogruppen in Lysinresten, wegfällt und somit der Synthesevorgang vereinfacht wird.

Von diesem Standpunkt her sind unter den erfindungsgemässen Acylpeptiden allgemein, sowie ihren weiter unten besonders hervorgehobenen Repräsentanten, solche bevorzugt, worin alle in Betracht kommenden Aminogruppen denselben Acylrest tragen.

Die vorliegende Erfindung betrifft insbesondere von Somatostatin und seinen Analogen abgeleitete Acylpeptide der allgemeinen Formel

$(A)_a$-$(B)_b$-$(C)_c$-D-Phe-trp-Lys(Ac)-Thr-E-$(Thr)_f$-$(G)_g$
3 4 5 6 7 8 9 10 11 12 13

(I)

worin

Ac ein an der freien Aminogruppe befindlicher Acylrest einer gegebenenfalls substituierten Alkancarbonsäure,

A der Rest der Teilformel

H-$Ala^1$-$Gly^2$-$Cys^3$- -$cys^{14}$-OH, Ac-$Ala^1$-$Gly^2$-$Cys^3$- -$cys^{14}$-OH, H-$Cys^3$- -$cys^{14}$-OH, Ac-$Cys^3$- -$cys^{14}$-OH oder Bmp- -$cys^{14}$-OH (wobei cys für L-Cys oder D-Cys und Bmp für den Desaminocystein-Rest steht), oder der Rest einer ω-Aminoniederalkancarbonsäure der Teilformel -NH-CH(R)-$(CH_2)_n$-CO- (wobei n eine ganze Zahl von 0 bis 6 bedeutet und R für Wasserstoff oder Carboxyl steht), welcher auch, wenn n = 2 und R Wasserstoff ist, durch einen cyclischen Hydrocarbylrest substituiert sein kann und in einem solchen Fall weiterhin als Gaba(Ar) bezeichnet wird,

B Lys, Lys(Ac) oder Lys(X) (wobei X eine ε-Aminoschutzgruppe ist),

C Asn, Ala oder His,

D Phe, oder, falls im Rest A keine schwefelhaltigen Aminosäurereste vorkommen, zusammen mit E der Rest -$Cys^6$- $Cys^{11}$- sein kann,

trp L-Trp, D-Trp oder ein analoger Rest, der im Indolkern, z.B. in der 5-Stellung, ein Halogen, insbesondere Fluor, trägt,

E Phe oder Tyr, oder zusammen mit D die oben angegebene Bedeutung hat,

G L-Ser, D-Ser oder der Rest einer sekundären α-Aminosäure mit höchstens 8 Kohlenstoffatomen ist, und

a, b, c, f und g unabhängig für 0 oder 1 stehen, sowie nicht-toxische Salze und pharmakologisch anwendbare Komplexe davon.

Die dem Acylrest Ac zugrunde liegende Alkancarbonsäure besitzt vorzugsweise nicht mehr als 18 Kohlenstoffatome, wenn sie unsubstituiert ist, und vorzugsweise nicht mehr als 8 Kohlenstoffatome,

wenn sie substituiert ist. Die Substituenten sind einerseits Hydroxyl-, Mercapto-, Niederalkylmercapto-, wie Methylmercapto-, Guanidino-, Carboxyl-, Carboxamido- und vor allem primäre Aminogruppen, oder eine an 2 verschiedenen Kohlenstoffatomen gebundene Iminogruppe, andererseits mono- oder bicyclische Hydrocarbyl- oder Heterocyclylreste, wie insbesondere Phenyl, p-Hydroxyphenyl, 1- oder 2-Naphthyl, 2-, 3- oder 4-Pyridyl, 2- oder 3-Indolyl, 2- oder 4-Imidazolyl, 2-, 4- oder 5-Thiazolyl, 2-Thienyl oder 2-Furyl. Die Säure kann einen oder mehrere gleichartige oder verschiedenartige Substituenten tragen, wobei die gesamte Anzahl der Kohlenstoffatome einschliesslich der kohlenstoffhaltigen Substituenten vorzugsweise höchstens 18 C-Atome beträgt. Besonders bevorzugt sind Acylreste, abgeleitet von einfach verzweigten oder insbesondere geradkettigen unsubstituierten Alkan-(mono oder di)-carbonsäuren, wobei die ersteren höchstens 18, die letzteren höchstens 9 Kohlenstoffatome besitzen, wie von der Essig-, Propion-, Butter-, Isobutter-, Valerian-, Isovalerian-, Capron-, Oenanth-, Undecan-, Laurin-, Myristin-, Palmitin- und Stearinsäure einerseits und der Malon-, Bernstein-, Glutar-, Adipin-, Pimelin- und Korksäure andererseits.

Besonders bevorzugt sind auch Acylreste abgeleitet von in der Natur, insbesondere als Peptid-Bausteine, vorkommenden $\alpha$-Aminosäuren der L-Reihe und deren nahe verwandten Analogen, wie insbesondere den Enantiomeren der «unnatürlichen» D-Reihe. Unter den bevorzugten $\alpha$-Aminosäuren kommen beispielsweise die folgenden ganz besonders in Betracht: Glycin, Alanin, Valin, Leucin, Isoleucin, Phenylalanin, Asparaginsäure, Glutaminsäure, Arginin, Lysin und Histidin, ferner $\beta$-Alanin, $\alpha$-Aminobuttersäure, $\gamma$-Aminobuttersäure, Norvalin, Isovalin, Norleucin und Ornithin, sowie auch Asparagin, Glutamin, Tyrosin, Tryptophan, Methionin, Threonin, Serin, und ganz besonders auch Prolin und Hydroxyprolin, bei denen die $\alpha$-Aminogruppe mit dem Alkylrest zu einem Ring zusammengeschlossen ist.

Die durch das Symbol X bezeichnete $\epsilon$-Aminoschutzgruppe des $Lys^4$-Restes hat die weiter unten angegebenen Bedeutungen. Sie unterscheidet sich von der oben charakterisierten Acylgruppe Ac grundsätzlich dadurch, dass sie unter Freigabe der Aminogruppe selektiv abspaltbar ist, wogegen die Acylgruppe Ac von der $\epsilon$-Aminogruppe ohne gleichzeitige Beeinträchtigung der peptidischen Amid-Bindungen nicht abzulösen ist.

Der als Gaba(Ar) bezeichnete Rest ist näher definiert durch die Formel

$$\begin{array}{c} \quad R_\gamma \quad R_\beta \quad R_\alpha \\ \quad | \quad\;\; | \quad\;\; | \\ -NH-CH-CH-CH-CO- \end{array}$$

worin jeweils eines der Symbole $R_\alpha$, $R_\beta$ und $R_\gamma$ ein unsubstituierter oder substituierter cyclischer Hydrocarbylrest Ar ist und die übrigen zwei für Wasserstoff stehen. Die dem Rest Gaba(Ar) entsprechende substituierte $\gamma$-Aminobuttersäure hat die Kurzbezeichnung H-Gaba(Ar)-OH.

Der cyclische Hydrocarbylrest Ar ist ein mono-, di- oder polycyclischer Cycloalkylrest oder ein entsprechender, mindestens einen aromatischen Ring enthaltender Arylrest mit höchstens 18, vorzugsweise höchstens 12 Ringkohlenstoffatomen. Unter den Cycloalkylresten sind bevorzugt solche mit 3- bis 8-, und vor allem 5- und/oder 6gliedrigen Ringen, wie beispielsweise Cyclopropyl, Cyclobutyl, Cycloheptyl, Cyclooctyl und ganz besonders Cyclopentyl und Cyclohexyl, ferner auch 1-Bicyclo[2,2,2]octyl, 2-Bicyclo[2,2,2]octyl, 2-Bicyclo[2,2,1]heptyl, 1- oder 2-Adamantyl, und 1- oder 2-Perhydronaphthyl, d.h. Bicyclo[4,4,0]decyl. Ein Arylrest ist in erster Linie ein Naphthyl, wie 1- oder 2-Naphthyl, ein entsprechendes teilweise hydriertes Naphthyl, wie insbesondere 1-, 2-, 5 oder 6-(1,2,3,4-Tetrahydronaphthyl), Phenyl, ein Anthryl, ein Fluorenyl und Azulenyl. Alle diese cyclischen Hydrocarbylreste können einen oder mehrere niederaliphatische Hydrocarbylreste, insbesondere Alkylreste mit höchstens 4 Kohlenstoffatomen, z.B. Methyl, Äthyl, Propyl, Isopropyl oder ein Butyl, und/oder weitere cyclische, insbesondere monocyclische Hydrocarbylreste, wie die oben definierten, tragen, wobei die Gesamtzahl der Kohlenstoffatome höchstens 18 Kohlenstoffatome beträgt. Als solche cyclische Hydrocarbylreste sind beispielsweise 4,4-Dimethylcyclohexyl, ein Tolyl, wie 2-, 3- oder 4-Tolyl und ein Biphenylyl, z.B. 4-Biphenylyl, zu nennen.

In ihrem aromatischen Teil können die cyclischen Hydrocarbylreste durch ein, zwei oder mehrere, gleiche oder verschiedene Substituenten substituiert sein, wie Halogen, z.B. Chlor, Brom, Jod und insbesondere Fluor, Phenoxy, Niederalkoxy, z.B. ein solches, das sich von einem der obengenannten Niederalkylreste mit höchstens 4 Kohlenstoffatomen ableitet, darunter vor allem Methoxy, ferner auch Nitro und Amino, insbesondere primäres Amino, Diniederalkylamino und Acylamino-, wie Niederalkanoylamino, z.B. Acetamino. Besonders bevorzugt sind durch die genannten Substituenten substituierte Phenylreste.

Der Rest Ar befindet sich in der $\alpha$-, $\gamma$- oder vorzugsweise $\beta$-Stellung der Kette der $\gamma$-Aminobuttersäure; demnach leiten sich besonders bevorzugte Reste der Formel Gaba(Ar) von den folgenden Buttersäuren: 4-Amino-3-phenyl-, 4-Amino-3-cyclohexyl-, 4-Amino-3-(2-naphthyl)- und vor allem 4-Amino-3-(1-naphthyl)-buttersäure und 4-Amino-3-(3-phenoxyphenyl)-buttersäure.

Die im Symbol G erwähnte sekundäre $\alpha$-Aminosäure mit höchstens 8 C-Atomen ist eine $\alpha$-Niederalkylaminoniederalkylcarbonsäure, in welcher die beiden Niederalkylreste durch eine C-C-Bindung, ein Sauerstoffatom, ein Schwefel(II)atom oder ein gegebenenfalls niederalkyliertes Stickstoffatom zusammen verbunden sein können, wobei jeder einzelne der Niederalkylreste höchstens 6 Kohlenstoffatome und alle zusammen höchstens 7 Kohlenstoffatome enthalten. Der Niederalkylrest, welcher dem Kohlenstoffgerüst der Carbonsäure zugrunde liegt, weist vorzugsweise mehr als ein C-Atom auf und ist insbesondere ein solcher, der in den natürlichen Aminosäuren vorkommt, wie Butyl, Isobutyl, Pentyl und insbesondere Äthyl oder Isopentyl. Das Niederalkyl, das als Substituent der Aminogruppe oder der Stickstoffbrücke vorkommt, ist vorzugsweise Methyl. Die C-C-Bindung, welche die beiden Niederalkylreste gegebenenfalls verbindet, ist vorzugsweise eine Einfachbindung. Die $\alpha$-Aminogruppe befindet sich vorzugsweise in einer solchen sterischen Konfiguration, die den natürlichen Aminosäuren, d.h. den L-Aminosäuren, entspricht. Es sind insbesondere die Reste solcher sekundären $\alpha$-Aminosäuren bevorzugt, die als in der Natur vorkommende Aminosäuren bekannt sind, wie vor allem L-Prolin, oder ihnen unmittelbar strukturell analog sind, wie einerseits 4-Oxa- und insbesondere 4-Thiaprolin der Formel

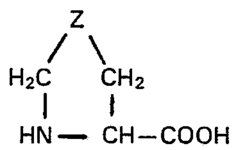

worin Z Sauerstoff oder Schwefel ist, und andererseits eine N-niederalkylierte, insbesondere N-methylierte, aliphatische Aminosäure, vor allem N-Methyl-L-leucin.

Unter den erfindungsgemässen Acylpeptiden sind diejenigen hervorzuheben, die sich von an sich bekannten, besonders wertvollen somatostatinartigen Grundpeptiden ableiten und durch spezifische Formeln charakterisiert sind, wie im Folgenden angegeben wird:

So liegt Somatostatin, D-Trp$^8$-Somatostatin, [D-Trp$^8$-D-Cys$^{14}$-]-Somatostatin, [(5-F)-D-Trp$^8$]-Somatostatin und Des-[Ala$^1$-Gly$^2$]-Somatostatin den Acylpeptiden der folgenden Formel IA zugrunde:

A'-Cys-B-Asn-Phe-Phe-trp-Lys(Ac)-Thr-Phe-Thr-

Ser-cys-OH (IA)

worin A' für H-Ala-Gly-, Ac-Ala-Gly-, H- oder Acsteht und Ac, B, trp und cys die eingangs angegebenen Bedeutungen haben; von Des-[Ala$^1$-Gly$^2$]-desamino-Cys$^3$-Somatostatin und seinen Strukturanalogen sind die Acylpeptide der Formel IB abgeleitet:

Bmp-B-C'-Phe-Phe-trp-Lys(Ac)-Thr-Phe-

Thr-G-Cys-OH (IB)

worin Ac, Bmp, B, trp und G die eingangs genannten Bedeutungen haben und C' für Asn oder His steht; von Oligopeptiden, in welchen eine oder mehrere der Aminosäuren in den Stellungen 1, 2, 4, 5, 12 und 13 des [D-Trp$^8$]-Somatostatins oder [D-Trp$^8$-D-Cys$^{14}$]-Somatostatins ausgelassen sind, leiten sich die Acylpeptide der Formel IC ab:

(Ala-Gly)$_a$-Cys-(B)$_b$-(Asn)$_c$-Phe-Phe-[D-Trp]-

Lys(Ac)-Thr-Phe-(Thr)$_f$-(Ser)$_g$-cys-OH (IC)

worin Ac, B und cys die eingangs angegebenen Bedeutungen haben und a, b, c, f und g unabhängig für 0 oder 1 stehen; von Analogen mit der (6-11)-Cystin-Brücke stammen die bicyclischen Acylpeptide der Formel I, worin A für den Rest der $\omega$-Aminoheptansäure, D und E zusammen für den Rest

-Cys$^6$- -Cys$^{11}$-, a für 1 und b, c, f und g für 0 stehen und welche der Formel ID

Cys-Phe-trp-Lys(Ac)-Thr-Cys (ID)

CO-(CH$_2$)$_6$-NH

entsprechen, worin Ac und trp die eingangs genannten Bedeutungen haben; und schliesslich liegen schwefelfreie Cyclopeptide mit Teilsequenzen von mindestens 6 und höchstens 11 der Aminosäuren des Somatostatin-Ringes den Acylpeptiden der Formel IE zugrunde:

(A'')$_a$-(B)$_b$-(C'')$_c$-Phe-Phe-trp-Lys(Ac)-Thr-

Phe-(Thr)$_f$-(Ser)$_g$ (IE),

worin a, b, c, f und g unabhängig für 0 oder 1 stehen, Ac, trp und B die eingangs genannten Bedeutungen haben, A'' den eingangs charakterisierten Rest -NH-CH(R)-(CH$_2$)$_n$-CO- bzw. Gaba(Ar) bedeutet und C'' für Asn oder Ala steht.

Unter den Acylpeptiden der Formel IE sind diejenigen besonders bevorzugt, worin Ac die eingangs definierten allgemeinen und besonders hervorgehobenen Bedeutungen hat, trp für D-trp steht, A'' einen $\omega$-Aminoniederalkancarbonsäure-Rest bedeutet, in welchem R für Wassestoff und n für eine ganze Zahl von 0 bis 3 steht, B für Lys, Lys(Ac) oder Lys(INOC) steht (wobei INOC das an der $\epsilon$-Aminogruppe befindliche Isonicotinyloxycarbonyl bedeutet), f und mindestens eines der Symbole a, b, c und g gleich 1 sind, wobei die übrigen unabhängig 0 oder 1 bedeuten, wie insbesondere diejenigen Verbindungen, worin a = 0 und b, c, f, g = 1, oder a, b = 0 und c, f, g = 1, oder a, b, c = 0 und f, g = 1, oder aber a, b, g = 0 und c, f = 1.

Unter den Acylpeptiden der Formel IE sind diejenigen ganz besonders bevorzugt, worin Ac die eingangs definierten allgemeinen und besonders hervorgehobenen Bedeutungen hat, trp für D-Trp steht, A'' den eingangs definierten Rest Gaba(Ar), insbesondere einen solchen mit dem Hydrocarbyl Ar in der $\beta$-Stellung, oder einen solchen $\omega$-Aminoniederalkylcarbonsäure-Rest darstellt, in welchem n eine ganze Zahl von 0 bis 6, insbesondere von 1 bis 3, und vor allem 2, und R Carboxyl und insbesondere Wasserstoff bedeutet, C'' für Ala und insbesondere für Asn steht, a gleich 1, c gleich 0 oder insbesondere 1 und b, f und g gleich 0 sind. Darunter sind wiederum Verbindungen hervorzuheben, worin im Rest A'' n = 5 und R Wasserstoff oder Carboxyl ist, a = 1 und b, c, f und g alle gleich 0 sind, und vor allem die Verbindungen der Formel

Asn-Phe-Phe-[trp$_o$]-Lys(Ac)-Thr-Phe-NH-

CH$_2$-CH(U)-CH$_2$-CO IF

worin $trp_o$ für D-Trp, welches auch in 5-Stellung Fluor tragen kann, steht, Ac die eingangs definierten allgemeinen und besonders hervorgehobenen Bedeutungen hat und U Wasserstoff oder den eingangs definierten Rest Ar, insbesondere den Phenyl-, Cyclohexyl-, 2-Naphthyl- und vor allem 1-Naphthyl- oder m-Phenoxyphenyl-Rest bedeutet.

Als Resultat der Substitution mit dem Rest Ar entsteht am β-Kohlenstoffatom der γ-Aminobuttersäure ein Asymmetriezentrum, welches das Vorliegen von jeweils zwei diastereomeren Formen des erfindungsgemässen Cyclopeptids zur Folge hat, die gewünschtenfalls voneinander getrennt, oder aber gemeinsam als Diastereomerengemisch für dieselben Verwendungszwecke eingesetzt werden können.

Ganz besonders bevorzugt sind Acylpeptide der oben stehenden Formeln I bis IF, in welchen Ac für einen Acylrest gemäss der Durchführungsbeispiele steht, oder welche das Grundgerüst eines der in den Beispielen gezeigten Acylpeptide haben. Vor allem sind dann die in den Beispielen gezeigten Acylpeptide bevorzugt.

Diejenigen der oben allgemein oder als bevorzugt charakterisierten Acylpeptide der Formel I, die eine freie Carboxylgruppe enthalten, können auch als Salze vorliegen, z.B. Natrium-, Kalium-, Calcium- oder Magnesiumsalze, oder auch Ammoniumsalze abgeleitet von Ammoniak oder einer physiologisch verträglichen organischen stickstoffhaltigen Base. Diejenigen der oben allgemein oder als bevorzugt charakterisierten Acylpeptide der Formel I, die eine freie Aminogruppe enthalten, können auch in Form von Salzen, und zwar von Säureadditionssalzen vorliegen. Als Säureadditionssalze kommen besonders physiologisch verträgliche Salze mit üblichen therapeutisch anwendbaren Säuren in Betracht; von den anorganischen Säuren sind die Halogenwasserstoffsäuren, wie die Chlorwasserstoffsäure, aber auch Schwefelsäure und Phosphor- bzw. Pyrophosphorsäure zu nennen, von den organischen Säuren sind es in erster Linie die Sulfonsäuren, z.B. die Benzol- oder p-Toluol-Sulfonsäure oder Niederalkansulfonsäuren, wie Methansulfonsäure, ferner auch Carbonsäuren, wie Essigsäure, Milchsäure, Palmitin- und Stearinsäure, Äpfelsäure, Weinsäure, Ascorbinsäure und Zitronensäure.

Die erfindungsgemässen Acylpeptide der Formel I können auch als Komplexe vorliegen. Unter Komplexen sind die in ihrer Struktur noch nicht völlig abgeklärten Verbindungen zu verstehen, die beim Zusatz gewisser anorganischer oder organischer Stoffe zu Peptiden entstehen und diesen eine verlängerte Wirkung verleihen. Solche Stoffe sind beispielsweise beschrieben für ACTH und andere adrenocorticotrop wirksame Peptide. Zu nennen sind z.B. anorganische Verbindungen, die sich von Metallen wie Calcium, Magnesium, Aluminium, Cobalt und insbesondere von Zink ableiten, vor allem schwerlösliche Salze, wie Phosphate, Pyrophosphate und Polyphosphate, sowie Hydroxyde dieser Metalle, ferner Alkalimetallpolyphosphate, z.B. «Calgon® N», «Calgon® 322», «Calgon® 188» oder «Polyron® B 12». Organische Stoffe, die eine Verlängerung der Wirkung hervorrufen, sind beispielsweise nicht antigene Gelatine-Arten, z.B. Polyoxygelatine, Polyvinylpyrrolidon und Carboxymethylcellulose, ferner Sulfonsäure- oder Phosphorsäureester von Alginsäure, Dextran, Polyphenolen und Polyalkoholen, vor allem Polyphloretinphosphat und Phytinsäure, sowie Polymerisate und Copolymerisate von basischen oder vor allem sauren Aminosäuren, z.B. Protamin oder Polyglutaminsäure.

Wenn nicht anders angegeben, beziehen sich die Kurzbezeichnungen der Aminosäurereste auf Reste der α-Aminosäuren der L-Reihe, welche in der Natur vorkommen.

Wenn nicht anders angegeben, bezeichnet der Begriff «nieder», wo immer er im Zusammenhang mit einem organischen Rest oder Verbindung vorkommt, einen solchen Rest oder Verbindung mit höchstens 7, vorzugsweise aber mit höchstens 4 Kohlenstoffatomen.

Die neuen erfindungsgemässen Acylpeptide weisen eine physiologische Wirkung auf, die im Grundcharakter der Wirkung des Somatostatins ähnlich ist. Sie können deshalb in ähnlichen therapeutischen Indikationen wie dieses, z.B. insbesondere zur Behandlung von Funktionsstörungen, in denen die Sekretion des Wachstumshormons oder des Glucagons übernormal hoch ist, wie bei Acromegalie oder Diabetes, mit Vorteil engewendet werden. Indem sie überdies noch Blutverluste im gastro-intestinalen Trakt hemmen, können sie auch in diesem Indikationsbereich mit Erfolg eingesetzt werden. Sie können auch als wertvolle Zwischenprodukte zur Herstellung anderer therapeutisch wertvoller Verbindungen dienen, z.B. solcher mit einem weiter modifizierten Acylrest Ac.

Die erfindungsgemässen Acylpeptide werden unter Anwendung von konventionellen, an sich bekannten Herstellungsverfahren der Peptidchemie erhalten.

So werden sie beispielsweise hergestellt, indem man ein entsprechendes Peptid mit freier ε-Aminogruppe des Lysinrestes in 9-Stellung, gegebenenfalls unter vorübergehendem Schutz vorhandener freier Hydroxylgruppen und/oder übriger freier Aminogruppen, acyliert. Die Acylierung erfolgt insbesondere dadurch, dass man ein Peptid der Formel

$$\ulcorner (A_o)_a\text{-}(B)_b\text{-}(C)_c\text{-D-Phe-trp-Lys-Thr}(Y_o)\text{-E-}$$

$$[\text{Thr}(Y_o)]_f\text{-}[G(Y_o)]_g \urcorner \qquad \text{(II)}$$

worin $A_o$ einen dem eingangs definierten Rest A entsprechenden Rest bedeutet, in welchem die α-Aminogruppe eines N-terminalen Aminosäurerestes eine α-Aminoschutzgruppe X' der unten näher definierten Bedeutung tragen kann, $Y_o$ ein am Sauerstoff befindlicher Wasserstoffatom oder Hydroxylschutzgruppe Y der unten näher definierten Bedeutung ist, und worin die übrigen Symbole die eingangs angegebenen Bedeutungen haben, mit einer Alkancarbonsäure $Ac_oOH$, worin $Ac_o$ einen dem eingangs definierten Acylrest Ac entsprechenden Rest bedeutet, in welchem vorhandene Amino- und Hydroxylgruppen Schutzgruppen X, X' bzw. Y tragen können, oder mit einem reaktionsfähigen Derivat einer solchen Säure behandelt und wenn erwünscht oder notwendig ist, im erhaltenen Produkt durch Abspaltung der Schutzgruppen X, X' und Y Aminogruppen bzw. Hydroxylgruppen freisetzt.

Der oben erwähnte Rest einer N-terminalen Aminosäure kommt nicht in allen Bedeutungen von A oder $A_o$ vor, sondern nur in denjenigen, die durch das Symbol A' dargestellt sind, und ist durch den Rest H-Ala$^1$- oder H-Cys$^3$- repräsentiert.

Die Bedeutung des Symbols X' entspricht umfanglich derjenigen der α-Aminoschutzgruppen, welche bei der Synthese der Peptidkette verwendet werden und weiter unten ausführlich beschrieben sind. Vorzugsweise werden gleichartige oder noch besser gleiche Schutzgruppen sowohl im Rest $A_o$ wie auch $Ac_o$ verwendet und im Anschluss an die Acylierungsreaktion gleichzeitig abgespalten.

Ein reaktionsfähiges Derivat der Säure $Ac_oOH$ ist beispielsweise ein Anhydrid, insbesondere ein symmetrisches Anhydrid der Formel $Ac_o$-O-$Ac_o$ oder ein cyclisches Anhydrid einer Dicarbonsäure, wie Succinanhydrid oder Glutaranhydrid, oder aber ein gemischtes Anhydrid mit einer anderen organischen Säure, z.B. mit Trifluoressigsäure, oder insbesondere mit einer anorganischen Säure, z.B. ein Säureazid oder Säurehalogenid, vor allem Säurechlorid. Ein reaktionsfähiges Säurederivat ist vorzugsweise ein aktivierter Ester, z.B. ein solcher, in welchem die Säure $Ac_oOH$ mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenzotriazol oder N-Hydroxypiperidin, oder aber mit einem N,N'-disubstituiertem Isoharnstoff, wie insbesondere N,N'-Dicyclohexylisoharnstoff, oder einer ähnlichen aus der Peptidchemie bekannten Aktivierungskomponente, vgl. Houben-Weyl: Methoden der organischen Chemie; 4. Auflage, Band 15/I und II, E. Wünsch (Herausgeber): Synthese von Peptiden (Georg Thieme Verlag, Stuttgart; 1974), verestert wird.

Die Acylierung erfolgt in an sich bekannter Weise, vorzugsweise in üblichen Lösungsmitteln, beispielsweise Dioxan, Tetrahydrofuran, Acetonitril, Pyridin, Dimethylformamid, Dimethylacetamid, Dimethylsulfoxid, N-Methylpyrrolidon, Hexamethylphosphortriamid, sowie Chloroform und Methylenchlorid, und in zweckmässigen Gemischen davon. Man kann auch mit einem Zusatz einer organischen Base, z.B. eines quaternären oder vor allem tertiären Amins, wie Triäthylamin, N-Äthylmorpholin oder N-Methylpiperidin, arbeiten, um die zu acylierende Aminogruppe in entprotonisierter Form zu erhalten. Die Reaktionstemperatur beträgt üblicherweise −20° bis +70°C, vorzugsweise etwa 0°C bei Raumtemperatur.

Als Acylierungsmittel sind im allgemeinen Aktivester deshalb vorteilhaft, weil sie Aminogruppen bevorzugt vor Hydroxylgruppen acylieren und somit den Schutz von Hydroxylgruppen praktisch überflüssig machen. Bei Dicarbonsäuren sind jedoch cyclische Anhydride bevorzugt, sofern sie vorhanden sind. Um eine unerwünschte O-Acylierung zu vermeiden, verwendet man üblicherweise nur ein Äquivalent des Acylierungsmittels für jede freie Aminogruppe des Ausgangsstoffes der Formel II.

Wenn es jedoch aus irgendeinem Grunde vorteilhafter ist, auf die selektive Acylierung zu verzichten, wie es insbesondere bei Umsetzung mit Säurechloriden der Fall sein kann, so nimmt man das Acylierungsmittel im Überschuss und setzt die mitacylierten Hydroxylgruppen nachträglich in gleicher konventioneller Weise wie die geschützten Hydroxylgruppen frei, insbesondere durch basische Hydrolyse, z.B. mit Natrium- oder Kaliumhydroxid in Anwesenheit von Wasser.

Die nachträgliche Abspaltung vorhandener Schutzgruppen richtet sich nach deren Art und erfolgt jeweils in einer an sich bekannten, konventionellen Weise, wie weiter unten ausführlich beschrieben. Dabei ist die Abspaltung vorhandener Hydroxylschutzgruppen Y, sowie von α-Aminoschutzgruppen X' in den Resten $A_o$ und $Ac_o$, eine obligatorische Massnahme; dagegen wird eine ε-Aminoschutzgruppe X im 4-ständigen Lysinrest nur gewünschtenfalls abgespalten.

Die erfindungsgemässen Acylpeptide kann man auch herstellen, indem man ein dem Acylpeptid entsprechendes lineares Peptid, gegebenenfalls unter vorübergehendem Schutz vorhandener freier Hydroxyl-, Carboxyl- und/oder Aminogruppen, cyclisiert. Ein entsprechendes lineares Peptid ist ein solches, das dieselben Aminosäuren in gleicher Reihenfolge wie das erfindungsgemässe cyclische Peptid hat, wobei aber eine Bindung zwischen zwei beliebigen benachbarten ringbildenden Aminosäuren unterbrochen ist und durch entsprechende endständige funktionelle Gruppen, die auch in einer aktivierten Form vorliegen können, ersetzt ist. Wenn der Ring an der amidischen Bindung zwischen zwei beliebigen nacheinanderfolgenden Aminosäuren unterbrochen ist, sind die endständigen Gruppen des linearen Peptids je eine Carboxyl- und Aminogruppe; wird aber der Ring zwischen zwei Cysteinresten, wie insbesondere zwischen denjenigen in 3- und 14-Stellung, unterbrochen, so wird eine disulfidische Bindung aufgelöst, und das entsprechende lineare Peptid hat als endständige Gruppen zwei freie oder funktionell abgewandelte Mercaptogruppen. Dem spezifischen Typ der endständigen Gruppen nach werden auch entsprechende Cyclisierungsverfahren angewendet.

Die erfindungsgemässen Verbindungen können durch Cyclisierung so hergestellt werden, dass man ein entsprechendes lineares Peptid der Formel

$$H\text{-}[I_a]\text{-}V \qquad (III),$$

worin $I_a$ einen der Formel I entsprechenden Rest darstellt, in welchem die amidische Bindung zwischen zwei beliebigen benachbarten Aminosäureresten des Peptidringes unterbrochen ist, und V für eine freie Hydroxylgruppe, eine durch eine Aktivierungsgruppe abgewandelte Hydroxylgruppe oder die Hydrazinogruppe $-NH-NH_2$ steht, cyclisiert, wobei gegebenenfalls vorhandene, an der Cyclisierungsreaktion nicht beteiligte Amino-, Carboxyl- und Hydroxylgruppen nach Bedarf in geschützter Form vorliegen und anschliessend freigesetzt werden.

Unter den linearen Peptiden der Formel III sind diejenigen bevorzugt, worin der Rest A als eine endständige Aminosäure im Rest [Ia] steht. Diese bevorzugten Ausgangsstoffe sind durch die Formeln

H-$(B)_b$-$(C)_c$-D-Phe-trp-Lys(Ac)-Thr-E-$(Thr)_f$-$(G)_g$-$(A)_a$-V (IIIa)

und insbesondere

H-$(A)_a$-$(B)_b$-$(C)_c$-D-Phe-trp-Lys(Ac)-Thr-E-$(Thr)_f$-$(G)_g$-V (IIIb)

charakterisiert, worin Ac, A, B, C, D, trp, E und G, sowie a, b, c, f und g die eingangs genannten und V die unmittelbar oben angegebenen Bedeutungen haben. Ganz besonders bevorzugt sind Verbindungen der Formeln IIIa und IIIb, worin im Rest A keine schwefelhaltige Aminosäure vorliegt.

Eine durch das Symbol V dargestellte funktionelle Gruppe ergänzt die Carbonylgruppe des C-terminalen Aminosäure-Restes und bildet zusammen mit ihr eine freie Carboxylgruppe, eine aktivierte Estergruppe, beziehungsweise die Carbazoylgruppe.

Die Aktivierungsgruppe, durch welche die Hydroxylgruppe abgewandelt ist, ist insbesondere eine solche, die den aktivierten Ester von N-Hydroxysuccinimid, 1-Hydroxybenzotriazol, N,N'-Dicyclohexylharnstoff, 2,4,5-Trichlorphenol, 2-Nitrophenol, 4-Nitrophenol, Pentachlorphenol oder Pentafluorphenol bildet, aber auch eine andere aus der Peptidchemie bekannte Aktivierungsgruppe dieser Art, vgl. Houben-Weyl, Band 15/II.

Die erfindungsgemässe Cyclisierung der linearen Peptide der Formel III erfolgt in an sich bekannter Weise mittels üblicher, zur Ausbildung der Amid-Bindung gebräuchlicher Kupplungsmethoden, wobei aber die peptidischen Ausgangsstoffe in einer sehr niedrigen Konzentration eingesetzt werden, um den Verlauf der Kupplung zugunsten der intramolekularen Cyclisierung auf Kosten der intermolekularen Polykondensation zu verschieben.

Die linearen Peptide werden mit Vorteil in einer etwa $1.10^{-4}$-molaren bis etwa $1.10^{-2}$-molaren, vorzugsweise einer etwa $1.10^{-3}$-molaren Konzentration eingesetzt, die einer Gewicht/Volumen-Konzentration von etwa 0,01 bis 1,0%, vorzugsweise 0,1% entspricht. Eine entsprechende Verdünnung kann im Reaktionsgemisch vom Anfang an eingestellt werden, oder aber durch langsames Eintropfen des Ausgangsstoffes und gegebenenfalls der übrigen Reagentien in das Reaktionsgemisch fortlaufend hergestellt werden.

Vorzugsweise erfolgt die Cyclisierung, indem man bei einer oben angegebenen Anfangskonzentration a) einen Ausgangsstoff der Formel III, worin V eine freie Hydroxylgruppe bedeutet, unter vorübergehendem Schutz vorhandener übriger Amino-, Carboxyl-, sowie Hydroxylgruppen mit einem Carbodiimid, gegebenenfalls in Anwesenheit einer Aktivester-bildenden Komponente, behandelt, oder b) einen Ausgangsstoff der Formel III, worin V eine zum aktivierten Ester abgewandelte Hydroxylgruppe darstellt und die endständige Aminogruppe in protonierter Form vorliegt, wobei mindestens die an der Cyclisierung nicht beteiligten Aminogruppen und Carboxylgruppen geschützt sind, mit einer organischen Base umsetzt, oder c) einen Ausgangsstoff der Formel III, worin V die Gruppe -$NHNH_2$ bedeutet, wobei mindestens die an der Cyclisierung nicht beteiligten Aminogruppen geschützt sind, zunächst unter sauren Bedingungen mit salpetriger Säure oder einem Niederalkylester davon behandelt und nachher bei einer oben erwähnten niedrigen Konzentration mit überschüssiger organischer Base cyclisiert.

Eine Carboxylgruppe wird in der weiter unten beschriebenen Weise durch eine Schutzgruppe W geschützt. Zum Schutz der Amino- und Hydroxylgruppen werden zweckmässig die Gruppen X, X' bzw. Y verwendet.

Die Cyclisierung führt man in geeigneten Lösungsmitteln durch; beispielsweise sind Dioxan, Tetrahydrofuran, Acetonitril, Pyridin, Dimethylformamid, Dimethylacetamid, Dimethylsulfoxid, N-Methylpyrrolidon, Hexamethylphosphortriamid, sowie auch Chloroform, Methylenchlorid oder Äthylacetat, sowie Gemische davon zu nennen.

Bei der Verfahrensvariante a) wird die Cyclisierung durch ein Carbodiimid, vorzugsweise N,N'-Dicyclohexylcarbodiimid, das man mit Vorteil im Überschuss anwendet, herbeigeführt; es ist anzunehmen, dass dabei der Ausgangsstoff der Formel III mit freier Carboxylgruppe primär in einen aktivierten Ester des Dicyclohexylisoharnstoffes (bzw. eines analogen Isoharnstoffes) übergeht und dieser *in situ* gebildete aktive Ester gleich weiterreagiert. Auf eine intermediäre Bildung eines aktiven Esters ist zweifellos die Zugabe einer Aktivester-bildenden Komponente als Hilfsreagens zurückzuführen; zu diesem Zwecke können in der Peptidchemie übliche Aktivester-bildende Komponenten dienen, wie insbesondere 2,4,5-Trichlorphenol, 2- oder 4-Nitrophenol, Pentachlor- und Pentafluorphenol, aber vor allem N-Hydroxyverbindungen, wovon als besonders vorteilhaft N-Hydroxysuccinimid, N-Hydroxypiperidin und vor allem 1-Hydroxybenzotriazol zu erwähnen sind. Die Arbeitstemperatur bei dieser Variante beträgt im allgemeinen 0-70°, vorzugsweise 35-55°.

Bei der Variante b), die mit fertigen Aktivestern, insbesondere den bereits hervorgehobenen, arbeitet, erfolgt die Cyclisierung spontan, als die endständige Aminogruppe durch die organische Base entprotonisiert wird. Die verwendeten Basen sind vorzugsweise quaternäre oder vor allem tertiäre Amine, z.B. Triäthylamin oder N-Äthylmorpholin. Vorzugsweise arbeitet man bei 10-30°, insbesondere bei Raumtemperatur.

Bei der Variante c) kann die erste Phase, d.h. die Bildung des Säureazids durch Behandlung mit salpetriger Säure oder einem Ester davon, mit Vorteil bei einer wesentlich höheren Konzentration der Ausgangsstoffe als die nachfolgende Cyclisierung erfolgen. Zweckmässig arbeitet man mit etwa einem Äquivalent eines Niederalkylnitrits, wie Äthyl-, Isoamyl- und insbesondere tert.-Butyl-nitrit, in salzsaurem Milieu bei Temperaturen von etwa —30° bis etwa —5°, vorzugsweise etwa —20°; ein geringer Überschuss an Nitrit ist zulässig. Danach wird die Lösung des gebildeten Azids nach der notwendigen Verdünnung bei einer Temperatur von etwa 0° bis

etwa 35° mittels einer überschüssigen organischen Base, z.B. einer der oben genannten, basisch gestellt und dadurch wie bei der Verfahrensvariante b zur spontanen Cyclisierung gebracht.

Im Spezialfall können die erfindungsgemässen Verbindungen, sofern sie ein Paar schwefelhaltiger Aminosäurereste, wie der von D- oder L-Cystein oder $\beta$-Mercaptopropionsäure, enthalten, dadurch hergestellt werden, dass man ein entsprechendes lineares Peptid der Formel

$T_o$-[$I_s$]-$T_o$ (IV)

worin $I_s$ einen der Formel I entsprechenden Rest darstellt, in welchem die disulfidische Bindung zwischen den schwefelhaltigen Aminosäureresten unterbrochen ist, und $T_o$ für Wasserstoff oder eine Mercaptoschutzgruppe T steht, zur Bildung der Disulfid-Brücke, gegebenenfalls unter vorangehender oder gleichzeitiger Abspaltung der Mercaptoschutzgruppen T oxidiert, und gegebenenfalls in geschützter Form vorhandene Amino-, Carboxyl- und/oder Hydroxylgruppen freisetzt.

Das lineare Peptid IV ist insbesondere ein solches der Formel

$T_o$-$A_1$-$(B)_b$-$(C)_c$-Phe-Phe-trp-Lys(Ac)-Thr-E-$(Thr)_f$-$(G)_g$-$A_2$-$T_o$ (IVa),

wobei $A_1$ für Ac-Ala-Gly-Cys-, H-Ala-Gly-Cys-, Ac-Cys-, H-Cys- oder Bmp-, $A_2$ für D oder L-Cys-OH steht, $T_o$ die unmittelbar oben angegebene Bedeutung hat und die übrigen Symbole die eingangs angegebenen Bedeutungen haben.

Das lineare Peptid IV ist aber auch ein solches der Formel

A''-$(B)_b$-$(C)_c$-Cys($T_o$)-Phe-Lys(Ac)-Thr-Cys($T_o$)-$(Thr)_f$-$(G)_g$ (IVb)

wobei A'' einen unter den Bedeutungen von A angegebenen schwefelfreien Aminosäurerest bedeutet, $T_o$ die unmittelbar oben angegebene Bedeutung hat und die übrigen Symbole die eingangs angegebenen Bedeutungen haben.

Die oxydative Cyclisierung erfolgt in der konventionellen, an sich allgemein bekannten Weise. Dabei kann man ein lineares Peptid der Formel IV oxydieren, in welchem alle Schutzgruppen vorher abgespalten wurden. Wenn jedoch das lineare Peptid der Formel IV in einer Form mit geschützten Amino-, Hydroxyl- und/oder Carboxylgruppen vorliegt, wie es in den meisten Fällen bei einer vorangehenden Synthese erhalten wird, ist es vorteilhaft, zuerst die Cyclisierung und erst dann die Abspaltung der Schutzgruppen (d.h. der Gruppen X, X', Y und W) durchzuführen. In einem solchen Fall schützt man vorzugsweise vorhandene Carboxylgruppen als tert.-Butylester, $\epsilon$-Aminogruppen durch die tert.-Butyloxycarbonylgruppe, die Hydroxylgruppen der Serin- und Threoninreste, sofern sie überhaupt geschützt werden, als tert.-Butyläther, und die Mercaptogruppen durch Trityl-, Acetaminomethyl-, p-Methoxybenzyl-, PCH- bzw. MPCH-, oder durch Tetrahydropyranylgruppen (Thp). Ausser Acetaminomethyl können alle diese funktionellen Gruppen in *einer* Stufe durch Einwirkung von Säuren (Acidolyse) abgespalten werden. Mercapto-Schutzgruppen vom Typ Trityl, Acetaminomethyl und Tetrahydropyranyl können aber, wenn erwünscht, unter Beibehaltung der Schutzgruppen vom tert.-Butyl-Typ mit Schwermetallsalzen, z.B. Mercuriacetat, und Schwefelwasserstoff selektiv abgespalten werden. Man erhält so ein lineares Peptid mit freien Mercaptogruppen, das man in einer an sich bekannten Weise, z.B. mit Jod, mit Dijodäthan in organischen Lösungsmitteln, oder mit Sauerstoff, insbesondere Luftsauerstoff, wie mit Luftsauerstoff in flüssigem Ammoniak, oxydativ cyclisieren kann. Noch vorteilhafter ist es, Mercaptogruppen durch Trityl-, Tetrahydropyranyl- oder Acylaminomethylgruppen zu schützen und diese unter gleichzeitiger Bildung der Disulfidbrücke mit Jod, z.B. in Methanol, Essigsäure oder insbesondere Dimethylformamid, zu entfernen, wobei die übrigen Schutzgruppen des genannten Typs erhalten bleiben und nachträglich abgespalten werden.

Die engere Auswahl der Schutzgruppen richtet sich nach dem spezifischen Zweck, wobei man insbesondere bei mehreren zu schützenden funktionellen Gruppen zweckmässige Kombinationen wählen muss.

Als $\epsilon$-Amino-Schutzgruppe X kann jede in der Peptid-Chemie übliche Amino-Schutzgruppe verwendet werden, wie sie in den entsprechenden Nachschlagwerken, z.B. in Houben-Weyl: Methoden der organischen Chemie; 4. Auflage, Band 15/I, E. Wünsch (Herausgeber): Synthese von Peptiden, (Georg Thieme Verlag, Stuttgart; 1974), zusammenfassend beschrieben werden.

So kann man z.B. reduktiv oder basisch abspaltbare Amino-Schutzgruppen verwenden, z.B. insbesondere die Benzyloxycarbonyl-Gruppe und Benzyloxycarbonyl-Gruppen, die im aromatischen Teil durch Halogenatome, Nitrogruppen, Niederalkoxygruppen und/oder Niederalkylreste substituiert sind, wie die p-Chlor- und p-Brombenzyloxycarbonyl-, p-Nitrobenzyloxycarbonyl-, p-Methoxybenzyloxycarbonyl-, p-Tolyloxycarbonyl-Gruppe, oder aber die Isonicotinyloxycarbonylgruppe, weiter auch Acylgruppen, wie p-Toluolsulfonyl, Benzolsulfenyl, o-Nitrobenzolsulfenyl bzw. auch Formyl, Trifluoracetyl oder Phthaloyl.

Ein vorteilhafte $\epsilon$-Amino-Schutzgruppe X ist eine Äthoxycarbonylgruppe, die in $\beta$-Stellung eine mit drei Kohlenwasserstoffresten substituierte Silylgruppe, wie Triphenylsilyl-, Dimethyl-butyl-silyl- oder vor allem Trimethylsilylgruppe, trägt. Eine solche $\beta$-(Trihydrocarbylsilyl-äthoxycarbonylgruppe, wie eine $\beta$-(Triniederalkylsilyl)-äthoxycarbonyl-, z.B. insbesondere die $\beta$-(Trimethylsilyl)-äthoxycarbonylgruppe, bildet mit der zu schützenden $\epsilon$-Aminogruppe eine entsprechende $\beta$-Trihydrocarbylsilyl-äthoxycarbonylaminogruppe (z.B. die $\beta$-Trimethylsilyläthoxycarbonylaminogruppe), welche unter den Bedingungen der sauren Hydrolyse und der Hydrogenolyse beständig ist, aber unter ganz spezifischen, sehr milden Bedingungen sich durch Einwirkung von Fluoridionen

abspalten lässt. In dieser Beziehung verhält sie sich analog wie die weiter unten als Carboxyl-Schutzgruppe beschriebene β-Silyläthylestergruppe. (Diese Ähnlichkeit muss bei der Synthese besonders berücksichtigt werden: bis auf Einzelfälle schliesst die Anwendung einer dieser Schutzgruppen die gleichzeitige Anwendung der anderen Schutzgruppe aus.) Weitere Einzelheiten sind weiter unten beim Schutz der Carboxylgruppe als β-Silyläthylester angegeben.

Ganz besonders bevorzugt sind acidolytisch abspaltbare Gruppen, wie in erster Linie die tert.-Butoxycarbonyl-Gruppe und analoge Gruppen, z.B. die tert.-Amyloxycarbonyl-, Isopropyloxycarbonyl-, Diisopropylmethoxycarbonyl-, Allyloxycarbonyl-, Cyclopentyloxycarbonyl-, Cyclohexyloxycarbonyl-, d-Isobornyloxycarbonyl- und Adamantyloxycarbonylgruppen, sowie auch Gruppen des Aralkyl-Typs, wie Benzhydryl und Triphenylmethyl (Trityl), oder bestimmte Aralkoxycarbonyl-Gruppen des 2-(p-Biphenylyl)-2-propyloxycarbonyl-Typs, die in der Schweizer Patentschrift 509 266 beschrieben sind.

Als Hydroxyl-Schutzgruppe Y können alle zu diesem Zwecke in der Peptid-Chemie gebräuchlichen Gruppen verwendet werden, vgl. das oben zitierte Werk (Houben-Weyl). Bevorzugt sind acidolytisch abspaltbare Gruppen, wie 2-Tetrahydropyranyl und ganz besonders tert.-Butyl, sowie auch tert.-Butoxycarbonyl. Ferner kann man aber auch reduktiv oder basisch abspaltbare Hydroxyl-Schutzgruppen verwenden, z.B. Benzyl- und Benzyloxycarbonylgruppen, die im aromatischen Teil durch Halogen, Nitro und/oder Niederalkoxy substituiert sein können, bzw. Niederalkanoylreste, wie Acetyl, oder Aroylreste, wie Benzoyl. Es ist auch möglich, unter Einhaltung gewisser einschränkender Massnahmen ohne Schutz der Hydroxylgruppen zu verfahren.

Als Carboxyl-Schutzgruppen W kann jede übliche zu diesem Zwecke gebräuchliche Gruppe verwendet werden, vgl. das oben zitierte Werk (Houben-Weyl). So werden Carboxylgruppen beispielsweise durch Hydrazidbildung oder durch Veresterung geschützt. Zur Veresterung geeignet sind z.B. niedere, gegebenenfalls substituierte Alkanole wie Methanol, Äthanol, Cyanmethylalkohol, 2,2,2-Trichloräthanol, Benzoylmethylalkohol oder insbesondere tert.-Butylalkohol, aber auch ein gegebenenfalls substituierter Benzylalkohol. Eine besonders vorteilhafte Kategorie der substituierten Alkanole sind Äthylalkohole, die in β-Stellung eine trisubstituierte Silylgruppe, wie Triphenylsilyl-, Dimethyl-butyl-silyl- oder vor allem Trimethylsilylgruppe, tragen. Wie z.B. im belgischen Patent Nr. 851 576 beschrieben ist, eignen sich diese Alkohole zum Schutze der Carboxylgruppen deshalb besonders gut, weil die entsprechenden β-Silyläthylester, z.B. β-(Trimethylsilyl)-äthylester, zwar die Stabilität üblicher Alkylester besitzen, sich jedoch unter milden Bedingungen durch Einwirkung von Fluoridionen unter Erhaltung aller anderen Schutzgruppen selektiv abspalten lassen.

Als Mercapto-Schutzgruppe T kann man alle zu diesem Zwecke in der Peptid-Chemie gebräuchlichen Gruppen verwenden, wobei die Mercaptogruppen insbesondere durch geeignete Acylierung oder Alkylierung geschützt werden. Zur Acylierung geeignet ist z.B. der Acetyl- oder Benzoylrest, ein Niederalkyl- (z.B. Äthyl-) -carbamoyl, oder eine gegebenenfalls, wie oben angegeben, substituierte Benzyloxycarbonyl-Gruppe (Carbobenzoxy-Gruppe). Zur Alkylierung geeignet sind z.B. der tert.-Butyl-, Isobutyloxymethyl-, Benzylthiomethyl- oder Tetrahydropyranylrest oder gegebenenfalls durch Halogen, Niederalkoxy oder Nitro substituierte Arylmethylreste wie Benzyl, p-Methoxybenzyl, Diphenylmethyl, Dimethoxybenzhydryl oder ganz besonders Trityl, sowie auch Phenylcyclohexyl (PCH), p-Methoxyphenylcyclohexyl (MPCH), Thienyl(2)-cyclohexyl u.a., vgl. Ber. *101*, 681 (1968). Sehr vorteilhaft ist auch ein Acylaminomethylrest der allgemeinen Formel $R_t$-CO-NH-$CH_2$-, worin $R_t$-CO- den Rest einer Carbonsäure bedeutet, vgl. Tetrahedron Letters *1968* (26), 3057 und die Deutsche Offenlegungsschrift 2 060 969. Der Acylrest $R_t$-CO- kann sich von einer aliphatischen, cycloaliphatischen, aromatischen, araliphatischen oder heterocyclischen Carbonsäure oder von einem Kohlensäuremonoderivat (wie einem Kohlensäuremonoester oder einer Carbaminsäure) ableiten. Durch das Symbol $R_t$ ist in erster Linie ein gegebenenfalls substituierter Niederalkylrest repräsentiert, z.B. Methyl-, Äthyl-, Propyl-, Isopropyl-, n-Butyl- oder tert.-Butylrest, der als Substituenten z.B. Chlor, Trifluormethyl oder die Nitrogruppe enthalten kann. Weiter bedeutet $R_t$ beispielsweise einen gegebenenfalls substituierten Cycloalkylrest mit 3-8, vorzugsweise 5-6, Ringatomen, wie den Cyclopentyl- oder Cyclohexylrest oder einen gegebenenfalls substituierten aromatischen oder araliphatischen, vorzugsweise monocyclischen Rest, vor allem einen gegebenenfalls substituierten Phenyl- oder Benzylrest, z.B. unsubstituiertes oder im Phenylrest durch Niederalkyl, Niederalkoxy, Halogen oder Nitro substituiertes Phenyl oder Benzyl, oder einen monocyclischen Heterocyclylrest, z.B. Thienyl oder Furyl. Besonders bevorzugt unter den Acylaminomethylgruppen ist die Acetylaminomethylgruppe.

Vorzugsweise werden die Schutzgruppen Y und W, sowie die weiter unten noch näher charakterisierte α-Aminoschutzgruppe X', so gewählt, dass sie unter ähnlichen Bedingungen abspaltbar sind; besonders bevorzugt sind dabei die bereits hervorgehobenen acidolytisch abspaltbaren Gruppen. Die Abspaltung aller dieser Schutzgruppen erfolgt dann vorteilhaft in einer einzigen Operation; es können jedoch auch Gruppen verschiedener Art verwendet werden und jede einzeln abgespalten werden.

Wenn jedoch im Endstoff der Formel I eine Schutzgruppe X, d.h. eine im $Lys^4$-Rest befindliche ε-Aminoschutzgruppe, erhalten bleiben soll, so sind die Reste X', Y und W so zu wählen, dass sie unter Erhaltung der Gruppe X abgespalten werden können.

Die Abspaltung der Schutzgruppen erfolgt in der allgemein bekannten Weise; die saure Hydrolyse (Acidolyse) wird z.B. mittels Trifluoressigsäure, Salzsäure oder Fluorwasserstoff, bei säureempfindlichen Schutzgruppen auch mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z.B. durch Hydrierung unter Palladium-Katalyse, entfernt. Die Isonicotinyloxycarbonylgruppe wird vorzugsweise durch Zink-Reduktion abgespalten.

Diejenigen erfindungsgemässen Endstoffe, die basische Gruppen enthalten, werden, je nach der Art der Isolierung, als Basen oder als Säureadditionssalze erhalten; diese können nachträglich in an sich bekannter Weise ineinander umgewandelt werden. Analogerweise können Endstoffe mit sauren Gruppen auch in Form von Salzen vorliegen, wobei beide Formen ineinander in bekannter Weise überführbar sind.

Auch die Bildung der oben erwähnten Komplexe erfolgt nach bekannten Methoden: Komplexe mit schwerlöslichen Metall-, z.B. Aluminium- oder Zinkverbindungen werden vorzugsweise in analoger Weise, wie für ACTH bekannt, z.B. durch Umsetzung mit einem löslichen Salz des betreffenden Metalls, z.B. Zinkchlorid oder Zinksulfat, und Ausfällung mit einem Alkalimetallphosphat und/oder -hydroxid hergestellt. Komplexe mit organischen Verbindungen vom Typ Polyoxygelatine, Carboxymethylcellulose, Polyvinylpyrrolidon, Polyphloretinphosphat, Polyglutaminsäure etc. werden durch Mischen dieser Substanzen mit dem Peptid in wässriger Lösung erhalten. In gleicher Weise können auch unlösliche Verbindungen mit Alkalimetallpolyphosphaten hergestellt werden.

Die Ausgangsstoffe der oben charakterisierten Formel III und IV, und, wenn nicht anders angegeben, auch die zu ihrer Synthese dienenden Zwischenprodukte sind neu und zum Teil auch zur Synthese anderer Somatostatin-Analogen, z.B. solcher mit analogen Aminosäure-Teilsequenzen, mit Vorteil anwendbar. Sie gehören, sowie auch ihre Herstellungsverfahren, zum Gegenstand der vorliegenden Erfindung. Sie werden nach an sich bekannten Methoden erhalten, indem man die zu ihrem Aufbau nötigen Aminosäuren bzw. kleinere Peptideinheiten unter Bildung von CO-NH-Bindungen in beliebiger zeitlicher Reihenfolge miteinander kondensiert, wobei nicht an der Reaktion teilnehmende funktionelle Gruppen intermediär geschützt werden können.

Bei der Herstellung dieser Ausgangsstoffe, wie auch aller benötigten Zwischenprodukte, kommen als Schutzgruppen für die endständigen $\alpha$-Amino- und Carboxylgruppen besonders die bei der Synthese langkettiger Peptide üblichen Schutzgruppen in Betracht, die z.B. durch Solvolyse oder Reduktion leicht und selektiv abgespalten werden können. Sie wurden vorstehend unter der Bezeichnung X' bzw. W bereits mehrmals erwähnt.

Als $\alpha$-Amino-Schutzgruppe X' sind beispielsweise zu nennen: gegebenenfalls, z.B. durch Halogen, Nitro, Niederalkyl oder Niederalkoxy, substituierte Di- oder Triarylniederalkylgruppen, wie Diphenylmethyl- oder Triphenylmethylgruppen, z.B. Benzhydryl, Trityl, Di-(p-methoxy)-benzhydryl, oder vor allem von der Kohlensäure sich ableitende hydrogenolytisch abspaltbare Gruppen, wie gegebenenfalls im aromatischen Rest durch Halogenatome, Nitrogruppen, Niederalkyl- oder Niederalkoxygruppen substituierte Benzyloxycarbonylgruppen, z.B. Benzyloxycarbonyl (d.h. Carbobenzoxy), p-Brom- oder p-Chlorbenzyloxycarbonyl, p-Nitrobenzyloxycarbonyl, p-Methoxybenzyloxycarbonyl; ferner auch 2-(p-Biphenylyl)-2-propyloxycarbonyl und ähnliche im Schweizer Patent 509 266 beschriebene Aryloxycarbonylgruppen. Dabei ist zu beachten, dass die $\alpha$-Aminoschutzgruppe X' selektiv unter Erhaltung der gegebenenfalls vorhandenen $\epsilon$-Aminoschutzgruppe X des 4-ständigen Lysinrestes abspaltbar sein muss. Es ist übrigens oft von Vorteil, wenn bei ihrer Abspaltung auch eine gegebenenfalls vorhandene Carboxyl- und Hydroxylschutzgruppe W bzw. Y unversehrt bleibt.

Die Carboxyl-Schutzgruppen für diesen Zweck sind dieselben, wie sie oben bei der entsprechenden Bedeutung des Symbols W besprochen wurden.

Diese Schutzgruppen können in bekannter Weise abgespalten werden. So kann man die Benzyloxycarbonylgruppe durch Hydrogenolyse, die N-Tritylgruppe mit Mineralsäuren, wie Halogenwasserstoffsäuren, z.B. Fluorwasserstoff oder vorzugsweise Chlorwasserstoff, oder einer organischen Säure, wie Ameisensäure, Essigsäure, Chloressigsäure oder Trifluoressigsäure, in wässrigem oder absolutem Trifluoräthanol als Lösungsmittel (vgl. deutsche Offenlegungsschrift DT 2 346 147) oder mit wässriger Essigsäure; die tert.-Butyloxycarbonylgruppe mit Trifluoressigsäure oder Salzsäure, die 2-(p-Biphenylyl)-isopropyloxycarbonylgruppe mit wässriger Essigsäure oder z.B. mit einem Gemisch von Eisessig, Ameisensäure (82,8%ig) und Wasser (7:1:2) oder nach dem Verfahren der DT 2 346 147 abspalten.

Die $\beta$-Silyläthylestergruppen werden vorzugsweise mit Fluoridionen-abgebenden Reagentien, z.B. Fluoriden quaternärer organischer Basen, wie Tetraäthylammoniumfluorid, abgespalten. Sie können aber auch, gleich wie die üblichen Alkylester, durch alkalische Hydrolyse, z.B. mittels Alkalimetallhydroxiden, -carbonaten oder -bicarbonaten, abgespalten werden oder durch Hydrazinolyse, z.B. mittels Hydrazinhydrat, in die entsprechenden Carbazoylgruppen umgewandelt werden. Zur Abspaltung von tert.-Butylestern verwendet man vorzugsweise Acidolyse, für Benzylester dann Hydrogenolyse.

Die zur Herstellung der Ausgangsstoffe der Formel III oder IV durchzuführende Kondensation der Aminosäure- und/oder Peptideinheiten erfolgt in an sich bekannter Weise, indem man vorzugsweise eine Aminosäure oder ein Peptid mit geschützter $\alpha$-Aminogruppe und gegebenenfalls aktivierter terminaler Carboxylgruppe (= aktive Komponente) an eine Aminosäure oder ein Peptid mit freier $\alpha$-Aminogruppe und freier oder geschützter z.B. veresterter terminaler Carboxylgruppe (= passive Komponente), anknüpft, im gebildeten Produkt die terminale Aminogruppe freisetzt und dieses Peptid, enthaltend eine freie $\alpha$-Aminogruppe und eine gegebenenfalls geschützte terminale Carboxylgruppe, wieder mit einer weiteren aktiven Komponente, d.h. einer Aminosäure oder einem Peptid mit aktivierter Carboxylgruppe und geschützter $\alpha$-Aminogruppe, umsetzt, usw. Die Carboxylgruppe kann beispielsweise durch Überführung in ein Säureazid, -anhydrid, -imidazolid oder einen aktivierten Ester, wie einen der weiter unten genannten, oder durch Reaktion mit einem

Carbodiimid, wie N,N'-Dicyclohexylcarbodiimid, gegebenenfalls unter Zusatz von N-Hydroxysuccinimid, einem unsubstituierten oder z.B. durch Halogen, Methyl oder Methoxy substituierten 1-Hydroxybenzotriazol oder 4-Hydroxybenzo-1,2,3-triazin-3-oxid (u.a., vgl. DT 1 917 690, DT 1 937 656, DT 2 202 613), oder insbesondere vom N-Hydroxy-5-norbornen-2,3-dicarboximid, oder N,N'-Carbonyldiimidazol aktiviert werden. Als die gebräuchlichste Kupplungsmethode ist die Carbodiimidmethode zu nennen, ferner auch die Azidmethode, die Methode der aktivierten Ester und die Anhydridmethode, sowie die Merrifield-Methode und die Methode der N-Carboxyanhydride oder N-Thiocarboxyanhydride.

Zur Bildung von aktivierten Estern, wie oben erwähnt sind, eignen sich z.B. gegebenenfalls durch elektronenanziehende Substituenten substituierte Phenole und Thiophenole wie Phenol, Thiophenol, Thiokresol, p-Nitrothiophenol, 2,4,5- und 2,4,6-Trichlorphenol, Pentachlorphenol, o- und p-Nitrophenol, 2,4-Dinitrophenol, p-Cyanophenol, weiter z.B. N-Hydroxysuccinimid, N-Hydroxyphthalimid und N-Hydroxypiperidin.

Bei einer besonders bevorzugten Herstellung der Peptide der Formel III und IV verwendet man als Kupplungsmethode die Carbodiimid-Methode mit N,N'-Dicyclohexylcarbodiimid in Anwesenheit von 1-Hydroxybenzotriazol. Die terminale Carboxylgruppe wird dabei in Form des $\beta$-(Trimethyl-silyl)-äthylesters geschützt, die $\alpha$-Aminogruppe der aktiven Komponente wird durch die Benzyloxycarbonylgruppe geschützt, die nach jedem Kupplungsschritt durch Hydrogenolyse abgespalten wird. Zum Schutz der $\epsilon$-Aminogruppe des 4-ständigen Lysinrestes wird die Acylierung mit der tert.-Butoxycarbonylgruppe und für die Hydroxylgruppe der Serin- und Threoninreste die Verätherung mit der tert.-Butylgruppe verwendet. Diese beiden Schutzgruppen können, wenn erwünscht, zuletzt in einem Schritt durch saure Hydrolyse, z.B. mittels Trifluoressigsäure, Salzsäure oder Fluorwasserstoff, abgespalten werden. Die $\epsilon$-Aminogruppe des Lysinrestes in 9-Stellung liegt in acylierter Form vor und benötigt deshalb keinen Schutz.

Die schwefelhaltigen Aminosäurereste (Bmp und cys) führt man vorzugsweise erst in den letzten Stadien der Synthese ein, da die Anwesenheit von Schwefel bekanntlich die Aktivität von Hydrierungskatalysatoren beeinträchtigen kann und somit die Anwendung der sonst sehr vorteilhaften hydrogenolytisch abspaltbaren Gruppen in Frage stellt. Die Mercaptogruppen in den besagten Säuren werden mit Vorteil durch die Tritylgruppen geschützt, welche für die Durchführung bevorzugter Verfahrensvarianten besonders geeignet sind.

Je nach Arbeitsweise erhält man die Verbindungen der Formel III und IV, je nach ihrem Charakter, in Form von Basen oder Säureadditionssalzen, oder aber von Säuren oder ihren Salzen. Aus den Säureadditionssalzen können in an sich bekannter Weise die Basen gewonnen werden. Von letzteren wiederum lassen sich durch Umsetzen mit Säuren, z.B. mit solchen, die die oben genannten Salze bilden, therapeutisch anwendbare Säureadditionssalze gewinnen. In ähnlicher Beziehung stehen auch Säuren und ihre Salze zueinander. Verbindungen, die sowohl eine freie Carboxylgruppe und eine basische Gruppe haben, können als innere Salze vorliegen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen der Verfahren, bei denen man von einer auf beliebiger Verfahrensstufe als Zwischenprodukt erhältlichen Verbindung ausgeht und die fehlenden Verfahrensschritte ausführt, oder wobei ein Ausgangsstoff unter den Reaktionsbedingungen gebildet oder in Form eines Derivats davon, zum Beispiel eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen.

Die vorliegende Erfindung betrifft ebenfalls pharmazeutische Präparate, welche Verbindungen der Formel I oder pharmazeutisch verwendbare Salze oder Komplexe davon enthalten. Diese Präparate können insbesondere in den oben angegebenen Indikationen Verwendung finden, wenn sie intraperitoneal, wie intravenös, intramuskulär oder subcutan, oder auch intranasal verabreicht werden. Die erforderliche Dosis hängt von der speziellen zu behandelnden Erkrankung, ihrer Schwere und von der Dauer der Therapie ab. Anzahl und Menge der Einzeldosen, sowie das Verabreichungsschema kann am besten anhand einer individuellen Untersuchung des jeweiligen Patienten bestimmt werden. Die Methode zur Bestimmung dieser Faktoren ist dem Fachmann geläufig. In der Regel liegt jedoch bei einer Injektion eine therapeutisch wirksame Menge einer solchen Verbindung im Dosisbereich von etwa 0,001 bis etwa 0,2 mg/kg Körpergewicht vor. Bevorzugt wird der Bereich von etwa 0,0015 bis etwa 0,15 mg/kg Körpergewicht und die Verabreichung erfolgt durch intravenöse Infusion oder subkutane Injektion. Dementsprechend enthalten pharmazeutische Präparate zur parenteralen Verabreichung in Einzeldosis-Form in Abhängigkeit von der Applikationsart pro Dosis etwa 0,08 bis etwa 15 mg einer der erfindungsgemässen Verbindungen. Neben dem Wirkstoff enthalten sie üblicherweise noch einen Puffer, z.B. einen Phosphatpuffer, der das pH zwischen etwa 3,5 und 7 halten soll, ferner Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z.B. 0,2-0,3% 4-Hydroxybenzoesäure-methylester oder -äthylester enthalten können. Soll in solchen Präparaten der Wirkstoff in Form eines Komplexes mit verlängerter Wirkungsdauer vorliegen, so kann dieser direkt durch Zusatz der komplexbildenden Komponenten zu einer Injektionslösung, die z.B. gemäss den oben erwähnten Massnahmen zubereitet werden, gebildet werden. Als Zusatz eignet sich z.B. 0,1-1,0 Gewichts-% eines Zink(II)-Salzes (z.B. Sulfats) in Verbindung mit

0,5-5,0 Gewichts-% Protamin (z.B. als Sulfat), bezogen auf das Gesamtvolumen der Injektionslösung; diese Zubereitung liegt als Lösung von pH 3,5 bis etwa 6,5 oder als Suspension von etwa pH 7,5 bis 8,0 vor.

Ein Präparat für die intranasale Verabreichung kann als eine wässrige Lösung oder Gelee, eine ölige Lösung oder Suspension, oder aber eine fetthaltige Salbe vorliegen. Ein Präparat in Form einer wässrigen Lösung erhält man z.B. dadurch, dass man den Wirkstoff der Formel I, oder ein therapeutisch anwendbares Säureadditionssalz davon, in einer wässrigen Pufferlösung von pH bis 7,2 löst und einen Isotonie erzeugenden Stoff zusetzt. Der wässrigen Lösung wird zweckmässig ein polymeres Haftmittel, z.B. Polyvinylpyrrolidon, und/oder ein Konservierungsmittel zugefügt. Die Einzeldosis beträgt etwa 0,08 bis etwa 15 mg, vorzugsweise 0,25 bis 10 mg, die in etwa 0,05 ml einer Lösung bzw. 0,05 g eines Gelees enthalten sind.

Eine ölige Applikationsform für die intranasale Verabreichung erhält man z.B. durch Suspendieren eines Peptids der Formel I oder eines therapeutisch anwendbaren Säureadditionssalzes davon in einem Öl, gegebenenfalls unter Zusatz von Quellmitteln, wie Aluminiumstearat, und/oder grenzflächenaktiven Mitteln (Tensiden), deren HLB-Wert («hydrophilic-lipophilic-balance») unter 10 liegt, wie Fettsäuremonoester mehrwertiger Alkohole, z.B. Glycerinmonostearat, Sorbitanmonolaurat, Sorbitanmonostearat oder Sorbitanmonoleat. — Eine fetthaltige Salbe erhält man z.B. durch Suspendieren des erfindungsgemässen Wirkstoffes in einer streichbaren Fettgrundlage, gegebenenfalls unter Zusatz eines Tensids vom HLB-Wert unter 10. Eine Emulsionssalbe erhält man durch Verreiben einer wässrigen Lösung des peptidischen Wirkstoffes in einer weichen, streichbaren Fettunterlage unter Zusatz eines Tensids, dessen HLB-Wert unter 10 liegt. Alle diese intranasale Applikationsformen können auch Konservierungsmittel enthalten. — Die Einzeldosen betragen etwa 0,08 bis etwa 15 mg, vorzugsweise 0,25 bis 10 mg, enthalten in etwa 0,05 bis etwa 0,1 g der Grundmasse.

Für die intranasale Verabreichung eignen sich weiter auch Inhalations- bzw. Insufflationspräparate, wie Insufflationskapseln, die den Wirkstoff in Form eines Puders mit der Atemluft zu insufflieren gestatten, oder Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise Hilfsmittel: Insufflationskapseln enthalten z.B. feste Trägerstoffe, wie Lactose; Aerosol- bzw. Sprayzubereitungen enthalten z.B. ein flüssiges Treibgas mit einem Siedepunkt unter der Raumtemperatur, sowie, wenn erwünscht, weitere Trägerstoffe, wie flüssige oder feste nicht-ionische oder anionische Tenside und/oder feste Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, die mittels einer geeigneten Verdichtungs- und Entspannungsvorrichtung nach Bedarf erzeugt wird.

Die Erfindung betrifft ebenfalls die Verwendung der neuen Verbindungen der Formel I und therapeutisch anwendbaren Säureadditionssalzen davon als pharmakologisch aktive Verbindungen, insbesondere in den eingangs angegebenen Indikationen, vorzugsweise in der Form von pharmazeutischen Präparaten. Die tägliche Dosis, die einem Warmblüter von etwa 70 kg verabreicht wird, beträgt von etwa 0,1 bis etwa 120 mg.

In den nachfolgenden Beispielen wird die Erfindung illustriert, jedoch durch diese nicht eingeschränkt. Temperaturen werden in Celsiusgraden angegeben; als Abkürzungen, z.B. zur Bezeichnung von Aminosäuren, Peptiden, Schutzgruppen etc., werden die üblichen, z.B. die in «Synthese von Peptiden» (Herausgeber: E. Wünsch), Bd XV der «Methoden der org. Chemie» (Houben-Weyl) (1974; G. Thieme, Stuttgart), zusammengestellten Kurzbezeichnungen verwendet. Insbesondere werden folgende Abkürzungen verwendet:

| | |
|---|---|
| Boc | — tert.-Butoxycarbonyl |
| But | — tert.-Butyl (als ätherbildende Gruppe) |
| OTmse | — 2-(Trimethylsilyl)-äthoxy (als esterbildende Gruppe) |
| Z | — Benzyloxycarbonyl (Carbobenzoxy) |
| DC | — Dünnschichtchromatographie |

In DC verwendet man, wenn nichts anderes angegeben ist, Kieselgel als Adsorbens und die folgenden Systeme als Laufmittel:

| | | |
|---|---|---|
| System | 52: | n-Butanol-Essigsäure-Wasser (71,5:7,5:21) |
| | 101: | n-Butanol-Pyridin-Essigsäure-Wasser (38:24:8:30) |
| | 104: | Chloroform-Methanol-17%iges wässriges Ammoniak (41:41:18) |
| | 111B: | n-Butanol-Pyridyn-25%iges wässriges Ammoniak-Wasser (40:24:6:30) |
| | 112A: | n-Butanol-Pyridin-Ameisensäure-Wasser (42:24:4:20) |
| | 151: | n-Butanol-Pyridin-Essigsäure-Wasser (38:20:6:24) |
| | 157: | Chloroform-Methanol-Wasser-Essigsäure (70:42:10:0,5) |
| | 157B: | (85:13:1,5:0,5) |
| | 157C: | (75:27:5:0,5) |
| | 157E: | (55:47:13:5) |

*Beispiel 1*

[$N^{\alpha}$-Acetyl-Ala$^1$, $N^{\epsilon}$-Acetyl-Lys$^4$, $N^{\epsilon}$-Acetyl-Lys$^9$]-Somatostatin

Ac Ac

Ac-Ala-Gly-Cys-Lys-Asn-Phe-Phe-Trp-Lys-Thr-

Phe-Thr-Ser-Cys-OH

(Ac = $CH_3CO$-)

50 mg Somatostatin (Peptidgehalt 82%, Rest Wasser und Essigsäure) in 175 $\mu$l Dimethylformamid werden mit 16 mg 4-Nitrophenylacetat und 12 $\mu$l Triäthylamin 2 Stunden bei Raumtemperatur gerührt. Das mit Äther-Hexan gefällte Produkt wird in Chloroform-Methanol (1:1) über eine Sephadex LH-20® - Säule filtriert und die nach DC reinen Fraktionen isoliert. Ausbeute: 44 mg.
DC: [Chloroform-Methanol-Wasser-Eisessig (55:47:13:0,5)]
$R_f$ 0,46

*Beispiel 2*
[D-Trp[8], N[ε]-*Acetyl*-Lys[9]-Gaba[12]]-cyclo-somatostatin(5-12)-octapeptid

Asn-Phe-Phe-(D-Trp)-Lys(Ac)-Thr-Phe-Gaba

(Ac = $CH_3CO$-)

100 mg Asn-Phe-Phe-(D-Trp)-Lys-Thr-Phe-Gaba (als Acetat) werden mit 15 $\mu$l N-Methylmorpholin und 20 mg p-Nitrophenylacetat in 1.0 ml Dimethylformamid gelöst und während 20 Stunden bei Raumtemperatur gehalten. Zur Aufbereitung wird das Reaktionsgemisch in 20 ml Äthylacetat aufgenommen und 3mal mit je 5 ml Wasser gewaschen. Die organische Phase wird nach dem Trocknen über Natriumsulfat im Vakuum eingedampft. Der Rückstand wird mit 5 ml Äther verrieben, abfiltriert und mit Äther gewaschen. Das Rohprodukt wird an einer Kieselgelsäule (70 g) mittels eines Chloroform-Methanol-Gemisches (mit graduell ansteigendem Anteil von 5 bis 15% Methanol) chromatographiert. Geeignete dünnschichtchromatographisch einheitliche Fraktionen werden vereinigt, im Vakuum eingedampft, in 20 ml tert.-Butanol gelöst und lyophilisiert.

DC: [$CHCl_3$-$CH_3OH$-$H_2O$ (14:6:1)] $R_f$ 0,65
[$CHCl_3$-$CH_3OH$ (85:15)] $R_f$ 0,20
[n-Butanol-Essigsäure-$H_2O$ (3:1:1)] $R_f$ 0,77

In analoger Weise werden aus demselben Peptid (als freie Base) unter Einhaltung von den sonstigen Bedingungen die folgenden N[ε]-Acyl-Lys[9]-Derivate erhalten, die als Zwischenprodukte für die Endstoffe des nachstehenden Beispiels 3 dienen:

a) Das N[ε]-(N-Benzyloxycarbonylglycyl)Lys[9]-Derivat (Ac = Z-Gly) wird mit 33 mg Z-Gly-p-nitrophenylester erhalten.

DC: [$CHCl_3$-$CH_3OH$-$H_2O$ (14:6:1)] $R_f$ 0,74
[$CHCl_3$-$CH_3OH$ (85:15)] $R_f$ 0,29

b) Das N[ε]-(N-Benzyloxycarbonylleucyl)Lys[9]-Derivat (Ac = Z-Leu) wird mit 38 mg Z-Leu-p-nitrophenylester erhalten.

DC: [$CHCl_3$-$CH_3OH$-$H_2O$ (14:6:1)] $R_f$ 0,80
[$CHCl_3$-$CH_3OH$ (85:15)] $R_f$ 0,35

c) Das N[ε]-(N-Benzyloxycarbonyl-phenylalanyl)-Lys[9]-Derivat (Ac = Z-Phe) wird mit 42 mg Z-Phe-p-nitrophenylester erhalten.

DC: [$CHCl_3$-$CH_3OH$-$H_2O$ (14:6:1)] $R_f$ 0,84
[$CHCl_3$-$CH_3OH$ (85:15)] $R_f$ 0,39

d) Das N[ε]-(N-Benzyloxycarbonyl-prolyl)-Lys[9]-Derivat (Ac = Z-Pro) wird mit 39 mg Z-Pro-p-nitrophenylester erhalten.

DC: System 157B $R_f$ 0,35

e) Das N[ε]-(N[α]-tert.-Butoxycarbonyl-N[ε]-benzyloxycarbonyl-lysyl)-Lys[9]-Derivat [Ac = Boc-Lys(Z)] wird mit 49 mg Boc-Lys(Z)-p-nitrophenylester erhalten.

DC: [$CHCl_3$-$CH_3OH$ (8:2)] $R_f$ 0,55

*Beispiel 3*
[D-Trp[8]-N[ε]-Glycyl-Lys[9]-Gaba[12]]-cyclo-somatostatin(5-12)-octapeptid

Asn-Phe-Phe-(D-Trp)-Lys(Ac)-Thr-Phe-Gaba

(Ac = Gly)

Eine Lösung von 78 mg

Asn-Phe-Phe-(D-Trp)-Lys(Z-Gly)-Thr-Phe-Gaba (siehe Beispiel 2a)

in 10 ml Methanol und 0,5 ml 1N-wässriger Essigsäure wird nach Zusatz von 10 mg Palladium-Kohle (10%) bei Raumtemperatur und Normaldruck während 10 Stunden hydriert. Zur Aufarbeitung wird vom Katalysator abfiltriert, das Filtrat im Vakuum eingedampft und der Rückstand in 20 ml tert.-Butanol gelöst und lyophilisiert.

In analoger Weise wird auch das entsprechende N[ε]-Leucyl- (Ac = Leu), N[ε]-Phenylalanyl- (Ac = Phe), N[ε]-Prolyl- (Ac = Pro) und N[ε]-(N[α]-tert.-Butoxycarbonyl)-lysyl-Derivat (Ac = Boc-Lys) hergestellt:

DC (Silicagel):

| System | $R_f$[Ac=Gly] | $R_f$[Ac=Leu] | $R_f$[Ac=Phe] | $R_f$[Ac=Pro] | $R_f$[Ac=Boc-Lys] |
|---|---|---|---|---|---|
| 101 | 0,60 | 0,69 | 0,71 | — | — |
| 111B | 0,62 | 0,80 | 0,80 | — | — |
| 112A | 0,58 | 0,70 | 0,82 | — | — |
| 151 | 0,59 | 0,70 | 0,70 | — | — |
| 157 | — | — | — | — | 0,65 |
| 157C | — | — | — | 0,73 | — |

Das letztgenannte $N^{\epsilon}$-($N^{\alpha}$-tert.-Butoxycarbonyl)-lysyl-Derivat (Ac = Boc-Lys) wird weiter zum entsprechenden Peptid mit freiem Lysyl-Rest, d.h. zum [D-$Trp^8$-$N^{\epsilon}$-Lysyl-$Lys^9$-$Gaba^{12}$]-cyclo-somatostatin-(5-12)-octapeptid der Formel

Asn-Phe-Phe-(D-Trp)-Lys(Lys)-Thr-Phe-Gaba

folgendermassen übergeführt:

100 mg Asn-Phe-Phe-(D-Trp)-Lys(Boc-Lys)-Thr-Phe-Gaba werden in 1,9 ml eines Gemisches von 89 Vol.% Trifluoressigsäure, 10 Vol.% Wasser und 1 Vol.% Thioglykolsäure unter Stickstoff bei 5° gelöst, die Lösung sofort auf 25° erwärmt, 25 Min. bei Raumtemperatur unter Stickstoff stehen gelassen. Das Produkt wird mit 20 ml Äther ausgefällt und aus tert.-Butanol lyophilisiert.

DC: System 101 $R_f$ 0,55

*Beispiel 4*
[D-$Trp^8$-$N^{\epsilon}$-Octanoyl-$Lys^9$-$Gaba^{12}$]-cyclo-somatostatin(5-12)-octapeptid

Asn-Phe-Phe-(D-Trp)-Lys(Ac)-Thr-Phe-Gaba

[Ac = $CH_3(CH_2)_6CO$-]

69 mg Asn-Phe-Phe-(D-Trp)-Lys-Thr-Phe-Gaba (als Acetat) in 0,25 ml Dimethylformamid werden mit 10 µl Triäthylamin und 23 mg Caprylsäureanhydrid (Octansäureanhydrid) versetzt und 15 Stunden bei Raumtemperatur belassen. Das Produkt wird mit Wasser gefällt und zur Reinigung in Chloroform-Methanol (1:1) über eine Sephadex LH-20® -Säule filtriert. Die nach DC reinen Fraktionen werden isoliert.
DC: [Chloroform-Methanol-Wasser-Eisessig (90:10:1:0,5)] $R_f$ 0,23

*Beispiel 5*
[D-$Trp^8$-$N^{\epsilon}$-Stearoyl-$Lys^9$-$Gaba^{12}$]-cyclo-somatostatin(5-12)-octapeptid

Asn-Phe-Phe-(D-Trp)-Lys(Ac)-Thr-Phe-Gaba

[Ac = $CH_3(CH_2)_{16}CO$-]

Eine Lösung von 66 mg Asn-Phe-Phe-(D-Trp)-Lys-Thr-Phe-Gaba (als Acetat) 26 mg Stearinsäurep-nitrophenylester und 9 µl Triäthylamin in 0,2 ml Dimethylformamid werden über Nacht bei Raumtemperatur belassen. Das mit Wasser gefällte Material wird anschliessend in Chloroform-Methanol (1:1) über eine Säule aus Sephadex LH-20® filtriert und die nach DC reinen Fraktionen isoliert.

DC: [Chloroform-Methanol-Wasser-Eisessig (75:26:5:0,5)] $R_f$ 0,18

*Beispiel 6*
[D-$Trp^8$-$N^{\epsilon}$-(β-Carboxypropionyl)-$Lys^9$-$Gaba^{12}$]-cyclo-somatostatin(5-12)-octapeptid

Asn-Phe-Phe-(D-Trp)-Lys(Ac)-Thr-Phe-Gaba

(Ac = HOOC-$CH_2CH_2$-CO-)

100 mg Asn-Phe-Phe-(D-Trp)-Lys-Thr-Phe-Gaba (als Acetat) werden in 0,8 ml Dimethylformamid gelöst und mit 9 µl N-Methylmorpholin und 8,2 mg Bernsteinsäureanhydrid versetzt. Nach 1 Stunde Reaktionszeit bei Raumtemperatur werden 5 ml (peroxidfreier) Äther zugefügt. Das ausgefallene, schmierige Rohprodukt wird zur Reinigung mit 5 ml Äther verrieben, in 5 ml tert.-Butanol gelöst und lyophilisiert.

| | | |
|---|---|---|
| DC: | [n-Butanol-Essigsäure-Wasser (3:1:1) | $R_f$ 0,65 |
| | System 52 | $R_f$ 0,63 |
| | System 104 | $R_f$ 0,60 |
| | System 111B | $R_f$ 0,50 |
| | System 157E | $R_f$ 0,58 |

*Beispiel 7*
[D-$Trp^8$-$N^{\epsilon}$-Acetyl-$Lys^9$-$Gaba^{12}$]-cyclo-somatostatin(5-12)-octapeptid

Asn-Phe-Phe-(D-Trp)-Lys(Ac)-Thr-Phe-Gaba

(Ac = $CH_3CO$)

Eine Lösung von 283 mg rohem H-Asn-Phe-Phe-(D-trp)-Lys(Ac)-Thr(But)-Phe-Gaba-OH (Stufe 7.7), 324 mg N-Hydroxybenzotriazol und 495 mg DCCI in 240 ml Dimethylformamid wird während 20 Stunden bei 50° gehalten. Zur Aufarbeitung wird das Lösungsmittel im Hochvakuum bei ca. 30° abgedampft und der Rückstand mit 10 ml Äthylacetat verrieben. Der ausgefallene Dicyclohexylharnstoff wird durch Abfiltrieren beseitigt, das Filtrat mit Äthylacetat auf 50 ml verdünnt, dreimal mit je 20 ml 1N wässeriger Oxalsäure und dann mit Wasser bis zur Neutralität gewaschen, über Natriumsulfat getrocknet und im Vakuum eingedampft. Das Rohprodukt wird zur Reinigung der Gegenstromverteilung im System Methanol-Wasser-Chloroform-Tetrachlorkohlenstoff (2700:675:900:1575 Vol.-Teile) über 430 Stufen unterzogen. Die in den Elementen 210 bis 254 enthaltenen Phasen (K = 1,16) werden vereinigt und im Vakuum eingedampft. Der Rückstand wird in 20 ml tert.-Butanol gelöst und lyophilisiert, womit ein dünnschichtchromatographisch einheitliches Material der Formel

Asn-Phe-Phe-(D-Trp)-Lys(Ac)-Thr(But)-Phe-Gaba (Ac = Acetyl)

resultiert.

DC: [Chloroform-Methanol (85:15)] $R_f$ 0,15
[Chloroform-Methanol-Wasser (14:6:1)] $R_f$ 0,70

Dieses geschützte Cyclopeptid (186 mg) wird bei 5° unter $N_2$ in 1,5 ml eines Gemisches von 89 Vol.% Trifluoressigsäure, 10 Vol.% Wasser und 1 Vol.% Thioglykolsäure gelöst, die Lösung sofort auf 25° erwärmt und nach 90 Minuten bei Raumtemperatur unter $N_2$ mit 15 ml Äther ausgefällt. Das resultierende Rohprodukt wird aus tert.-Butanol lyophilisiert.

Die erhaltene Titelverbindung ist in drei Systemen dünnschichtchromatographisch mit dem Produkt des Beispiels 2 identisch.

Das als Ausgangsstoff verwendete lineare Octapeptid wird folgendermassen erhalten:

*Stufe 7.1*
*Z-(D-trp)-Lys(Ac)-OH*

Zu einer Lösung von 33,84 g Z-(D-Trp)-OH und 17,42 g 8-Hydroxychinolin in 50 ml Acetonitril wird bei 0° bis 5° eine Lösung von 21,87 g DCCI in 100 ml Acetonitril während 45 Minuten zugetropft. Nach weiteren 30 Minuten bei 5° wird der ausgefallene Dicyclohexylharnstoff durch Filtration und Auswaschen mit 50 ml Acetonitril entfernt. Das Filtrat versetzt man mit einer Lösung von 20,70 g H-Lys(Ac)-OH in 25,9 ml 4,25N-Kalilauge und 80 ml Acetonitril und lässt während 15 Stunden bei Raumtemperatur stehen. Zur Aufarbeitung wird das Reaktionsgemisch im Vakuum eingedampft, der Rückstand in 1 Liter Äthylacetat aufgenommen, dreimal mit je 200 ml 1N-Salzsäure bei 0° und dreimal mit je 200 ml Wasser gewaschen, die organische Phase über Natriumsulfat getrocknet und im Vakuum eingedampft. Das erhaltene braune Öl wird in 150 ml Chloroform gelöst und unter starkem Rühren in 1,5 Liter Hexan getropft. Die flockige, klebrige Ausfällung wird abfiltriert, mit 500 ml Hexan gewaschen und im Vakuum getrocknet. Zur weiteren Reinigung wird das Material in 150 ml Tetrachlorkohlenstoff-Äthylacetat (6:4 Vol.-Teile) gelöst und auf einer Kieselgelsäule mit diesem Lösungsmittel-Gemisch chromatographiert. Geeignete dünnschichtchromatographisch einheitliche Fraktionen werden im Vakuum eingedampft, wodurch das reine Produkt in Form einer schaumartigen Masse erhalten wird.
DC: [Chloroform-Methanol-Wasser (14:6:1)] $R_f$ 0,45

*Stufe 7.2*
*Z-(D-Trp)-Lys(Ac)-Thr(But)-Phe-OTmse*

Eine Lösung von 7,24 g rohem (87%ig gemäss Titrierung) H-Thr(But)-Phe-OTmse und 7,58 g Z-(D-Trp)-Lys(Ac)-OH (Stufe 7.1) in 100 ml Dimethylformamid wird bei 5° mit 2,28 g N-Hydroxybenzotriazol und 3,38 g DCCI versetzt und das Reaktionsgemisch während 1 Stunde bei 5° und weiterer 15 Stunden bei Raumtemperatur gehalten. Zur Aufarbeitung wird der ausgefallene Dicyclohexylharnstoff durch Abfiltrieren entfernt und das Filtrat im Hochvakuum eingedampft. Der Rückstand wird zweimal aus Äthylacetat-Petroläther umgefällt und im Vakuum getrocknet.
DC: [Chloroform-Äthylacetat (1:1)] $R_f$ 0,18
[Toluol-Aceton (1:1)] $R_f$ 0,50

*Stufe 7.3*
*H-(D-Trp)-Lys(Ac)-Thr(But)-Phe-OTmse*

Eine Lösung von 5,00 g Z-(D-Trp)-Lys(Ac)-Thr-(But)-Phe-OTmse (Stufe 7.2) in 300 ml Methanol wird nach Zugabe von 0,50 g Palladium-Kohle (10%) bei Raumtemperatur und Normaldruck während 5 Stunden hydriert. Zur Aufarbeitung wird vom Katalysator abfiltriert und der nach dem Eindampfen des Filtrates verbleibende Rückstand sofort in Stufe 7.4 weiter eingesetzt.

*Stufe 7.4*
*Z-Asn-Phe-Phe-(D-Trp)-Lys(Ac)-Thr(But)-Phe-OTmse*

Eine Lösung von 632 mg Z-Asn-Phe-Phe-OH und 803 mg H-(D-Trp)-Lys(Ac)-Thr(But)-Phe-OTmse (Stufe 7.3) in 5 ml Dimethylformamid wird mit 210 mg N-Hydroxybenzotriazol und 276 mg DCCI versetzt und 15 Stunden bei Raumtemperatur belassen. Zur Aufarbeitung wird der ausgefallene Dicyclohexylharnstoff durch Abfiltrieren beseitigt und das Filtrat im Hochvakuum eingedampft. Der ölige Rückstand wird mit 5 ml Methanol verrieben und abgesaugt. Das ungelöste Material wird zur Reinigung nochmals mit 5 ml Methanol bei 50° verrieben, abgesaugt, mit Methanol gewaschen und im Vakuum getrocknet. Das Produkt ist gemäss DC einheitlich.
DC: [Chloroform-Methanol (85:15)] $R_f$ 0,78
[Chloroform-Methanol-Wasser (14:6:1)] $R_f$ 0,82

*Stufe 7.5*
*Z-Asn-Phe-Phe-(D-Trp)-Lys(Ac)-Thr(But)-Phe-OH*

900 mg Z-Asn-Phe-Phe-(D-Trp)-Lys(Ac)-Thr(But)-Phe-OTmse (Stufe 7.4) wird in 23 ml einer frisch hergestellten, wasserfreien 0,15N-Tetraäthylammoniumfluorid-Lösung in Dimethylformamid gelöst und 30 Minuten bei 25° gehalten. Nach dem Abkühlen auf 5° wird das Reaktionsgemisch unter gutem Umrühren mit 0,68 ml 1N wässeriger Salzsäure versetzt und das Produkt durch Zugabe von 70 ml Wasser gefällt. Das abfiltrierte Material wird mit 5 ml Wasser gewaschen, im Vakuum über Phosphorpentoxid getrocknet und direkt in Stufe 7.6 eingesetzt.

*Stufe 7.6*
*Z-Asn-Phe-Phe-(D-Trp)-Lys(Ac)-Thr(But)-Phe-Gaba-OBzl*

Ein Gemisch von 411 mg Z-Asn-Phe-Phe-(D-Trp)-Lys(Ac)-Thr(But)-Phe-OH (Stufe 7.5) und 82 mg Gaba-benzylester-p-toluolsulfonat in 2 ml Dimethylformamid wird mit 61 mg 1-Hydroxybenzotriazol und 93 mg DCCI versetzt und 20 Stunden bei Raumtemperatur belassen. Zur Aufarbeitung wird das Gemisch mit 15 ml eiskaltem Methanol versetzt und abfiltriert. Der gewonnene Feststoff wird zur weiteren Reinigung mit 5 ml warmem Methanol während 10 Minuten verrührt, die Suspension auf 0° abgekühlt, das reine Produkt abfiltriert und im Vakuum getrocknet.
DC: [Chloroform-Methanol (85:15)] $R_f$ 0,75

*Stufe 7.7*
*H-Asn-Phe-Phe-(D-Trp)-Lys(Ac)-Thr(But)-Phe-Gaba-OH*

Eine Lösung von 400 mg Z-Asn-Phe-Phe-(D-Trp)-Lys(Ac)-Thr(But)-Phe-Gaba-OBzl (Stufe 7.6) in 25 ml Dimethylformamid wird nach Zugabe von 50 mg Palladium-Kohle (10%) während 6 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 25 ml peroxidfreiem Äther ausgefällt, abfiltriert und im Vakuum getrocknet. Das Rohprodukt wird ohne weitere Reinigung der Endstufe (Cyclisierung) unterzogen.

**Patentansprüche für die Vertragsstaaten:**
BE, CH, DE, FR, GB, IT, LI, LU, NL, SE

1. Ein Acylpeptid, das von sich von Somatostatin oder einem wirkungsanalogen Derivat davon, dessen Aminosäuresequenz durch Auslassen einzelner Aminosäuren und/oder deren Austausch gegen andere Aminosäuren modifiziert ist, ableitet und dadurch gekennzeichnet ist; dass darin die ε-Aminogruppe des Lysinrestes in 9-Stellung, und gegebenenfalls auch die ε-Aminogruppe des Lysinrestes in 4-Stellung und/oder die N-terminale α-Aminogruppe, den Acylrest einer gegebenenfalls substituierten Alkancarbonsäure trägt, seine Salze und Komplexe davon.

2. Ein Acylpeptid gemäss Anspruch 1 der allgemeinen Formel

$$\underline{\overline{(A)_a\text{-}(B)_b\text{-}(C)_c\text{-D-Phe-trp-Lys(Ac)-Thr-E-}(Thr)_f\text{-}(G)_g}}$$

3 4 5 6 7 8 9 10 11 12 13

(I)

worin

Ac ein an der freien Aminogruppe befindlicher Acylrest einer gegebenenfalls substituierten Alkancarbonsäure,

A der Rest der Teilformel

H-$Ala^1$-$Gly^2$-$Cys^3$- -$cys^{14}$-OH, Ac-$Ala^1$-$Gly^2$-$Cys^3$- -$cys^{14}$-OH, H-$Cys^3$- -$cys^{14}$-OH, Ac-$Cys^3$- -$cys^{14}$-OH oder Bmp- -$cys^{14}$-OH

(wobei cys für L-Cys oder D-Cys und Bmp für den Desaminocystein-Rest steht), oder der Rest einer ω-Aminoniederalkancarbonsäure der Teilformel -NH-CH(R)-$(CH_2)_n$-CO- (wobei n eine ganze Zahl von 0 bis 6 bedeutet und R für Wasserstoff oder Carboxyl steht), welcher auch, wenn n = 2 und R Wasserstoff ist, durch einen cyclischen Hydrocarbylrest substituiert sein kann und in einem solchen Fall durch das Symbol Gaba(Ar) bezeichnet wird,

B Lys, Lys(Ac) oder Lys(X) (wobei X eine ε-Aminoschutzgruppe ist),

C Asn, Ala oder His,

D Phe, oder, falls im Rest A keine schwefelhaltigen Aminosäurereste vorkommen, zusammen mit E der Rest -$Cys^6$- $Cys^{11}$- sein kann,

trp L-Trp, D-Trp oder ein analoger Rest, der im Indolkern, ein Halogen trägt,

E Phe oder Tyr, oder zusammen mit D die oben angegebene Bedeutung hat,

G L-Ser, D-Ser oder der Rest einer sekundären α-Aminosäure mit höchstens 8 Kohlenstoffatomen ist, und

a, b, c, f und g unabhängig für 0 oder 1 stehen, sowie nicht-toxische Salze und pharmakologisch anwendbare Komplexe davon.

3. Ein Acylpeptid gemäss Anspruch 2 der allgemeinen Formel

A'-Cys-B-Asn-Phe-Phe-trp-Lys(Ac)-Thr-Phe-Thr-Ser-cys-OH (IA)

worin A' für H-Ala-Gly-, Ac-Ala-Gly-, H- oder Acsteht und Ac, B, trp und cys die im Anspruch 2 angegebenen Bedeutungen haben.

4. Ein Acylpeptid gemäss Anspruch 2 der allgemeinen Formel

Asn-Phe-Phe-[$trp_o$]-Lys(Ac)-Thr-Phe-NH-$CH_2$-CH(U)-$CH_2$-CO IF

worin $trp_o$ für D-Trp oder D-(5F)Trp steht, Ac die im Anspruch 2 definierten Bedeutungen hat und U Wasserstoff oder einen gegebenenfalls substituierten cyclischen Hydrocarbylrest Ar bedeutet.

5. Ein Acylpeptid gemäss Anspruch 4, worin U Wasserstoff und Ac den Rest einer in der Natur vorkommenden α-Aminosäure oder eines nahe verwandten Analogen davon bedeutet und trp die im Anspruch 4 angegebene Bedeutung hat.

6. Ein Acylpeptid gemäss Anspruch 4 der Formel IF, worin $trp_o$ für D-Trp, U für Wasserstoff und Ac für Glycyl, Leucyl oder Phenylalanyl steht.

7. Ein Acylpeptid gemäss Anspruch 4 der Formel IF, worin $trp_o$ für D-Trp, U für Wasserstoff und Ac für Prolyl steht.

8. Ein Acylpeptid gemäss Anspruch 4 der Formel IF, worin $trp_o$ für D-Trp, U für Wasserstoff und Ac für $N^\alpha$-tert.-Butoxycarbonyl-lysyl oder Lysyl steht.

9. Verfahren zur Herstellung von Acylpeptiden, die sich von Somatostatin oder einem wirkungsanalogen Derivat davon, dessen Aminosäuresequenz durch Auslassen einzelner Aminosäuren und/oder deren Austausch gegen andere Aminosäuren modifiziert ist, ableiten und in welchen die ε-Aminogruppe des Lysinrestes in 9-Stellung, und gegebenenfalls auch die ε-Aminogruppe des Lysinrestes in 4-Stellung und/oder die N-terminale α-Aminogruppe, den Acylrest einer gegebenenfalls substituierten Alkancarbonsäure trägt, sowie Salzen und Komplexen davon, dadurch gekennzeichnet, dass man ein entsprechendes Peptid mit freier ε-Aminogruppe des Lysinrestes in 9-Stellung, gegebenenfalls unter vorübergehendem Schutz vorhandener freier Hydroxygruppen oder übriger freier Aminogruppen, acyliert, oder ein dem Acylpeptid entsprechendes lineares Peptid, gegebenenfalls unter vorübergehendem Schutz vorhandener freier Hydroxyl-, Carboxyl- und/oder Aminogruppen, cyclisiert, und, wenn erwünscht, in Form von Salz erhaltene Verbindungen in die entsprechende freie Form oder eine erhaltene freie Verbindung in ein entsprechendes Salz umwandelt, und/oder, wenn erwünscht, eine erhaltene Verbindung in ihr Komplex überführt.

10. Ein pharmazeutisches Präparat enthaltend ein in einem der Ansprüche 1-6 definiertes Acylpeptid.

11. Ein pharmazeutisches Präparat enthaltend ein in Anspruch 7 oder 8 definiertes Acylpeptid.

12. Ein in einem der Ansprüche 1-6 definiertes Acylpeptid als Antidiabeticum.

13. Ein in Anspruch 7 oder 8 definiertes Acylpeptid als Antidiabeticum.

**Patentansprüche für den Vertragsstaat:**
AT

1. Verfahren zur Herstellung von Acylpeptiden, die sich von Somatostatin oder einem wirkungsanalogen Derivat davon, dessen Aminosäuresequenz durch Auslassen einzelner Aminosäuren und/oder deren Austausch gegen andere Aminsäuren modifiziert ist, ableiten und, als kennzeichnendes Merkmal, an der $\epsilon$-Aminogruppe des Lysinrestes in 9-Stellung, und gegebenenfalls auch an der $\epsilon$-Aminogruppe des Lysinrestes in 4-Stellung und/oder der N-terminale $\alpha$-Aminogruppe, den Acylrest einer gegebenenfalls substituierten Alkancarbonsäure tragen, sowie von Salzen und Komplexen davon, dadurch gekennzeichnet, dass man ein entsprechendes Peptid mit freier $\epsilon$-Aminogruppe des Lysinrestes in 9-Stellung, gegebenenfalls unter vorübergehendem Schutz vorhandener freier Hydroxygruppen oder übriger freier Aminogruppen, acyliert, oder ein dem Acylpeptid entsprechendes lineares Peptid, gegebenenfalls unter vorübergehendem Schutz vorhandener freier Hydroxyl-, Carboxyl- und/oder Aminogruppen, cyclisiert, und, wenn erwünscht, in Form von Salz erhaltene Verbindungen in die entsprechende freie Form oder eine erhaltene freie Verbindung in ein entsprechendes Salz umwandelt, und/oder, wenn erwünscht, eine erhaltene Verbindung in ihr Komplex überführt.

2. Verfahren gemäss Anspruch 1 zur Herstellung von Acylpeptiden der allgemeinen Formel

$(A)_a$-$(B)_b$-$(C)_c$-D-Phe-trp-Lys(Ac)-Thr-E- $(Thr)_f$-$(G)_g$
3 4 5 6 7 8 9 10 11 12 13

(I)

worin

Ac ein an der freien Aminogruppe befindlicher Acylrest einer gegebenenfalls substituierten Alkancarbonsäure,

A der Rest der Teilformel

H-Ala$^1$-Gly$^2$-Cys$^3$- -cys$^{14}$-OH, Ac-Ala$^1$-Gly$^2$-Cys$^3$- -cys$^{14}$-OH, H-Cys$^3$- -cys$^{14}$-OH, Ac-Cys$^3$- -cys$^{14}$-OH oder Bmp- -cys$^{14}$-OH

(wobei cys für L-Cys oder D-Cys und Bmp für den Desaminocystein-Rest steht), oder der Rest einer $\omega$-Aminoniederalkancarbonsäure der Teilformel -NH-CH(R)-$(CH_2)_n$-CO- (wobei n eine ganze Zahl von 0 bis 6 bedeutet und R für Wasserstoff oder Carboxyl steht), welcher auch, wenn $n = 2$ und R Wasserstoff ist, durch einen cyclischen Hydrocarbylrest substituiert sein kann und in einem solchen Fall durch das Symbol Gaba(Ar) bezeichnet wird,

B Lys, Lys(Ac) oder Lys(X) (wobei X eine $\epsilon$-Aminoschutzgruppe ist),

C Asn, Ala oder His,

D Phe, oder, falls im Rest A keine schwefelhaltigen Aminosäurereste vorkommen, zusammen mit E der Rest -Cys$^6$- Cys$^{11}$- sein kann,

trp L-Trp, D-Trp oder ein analoger Rest, der im Indolkern, z.B. in der 5-Stellung, ein Halogen, insbesondere Fluor, trägt,

E Phe oder Tyr, oder zusammen mit D die oben angegebene Bedeutung hat,

G L-Ser, D-Ser oder der Rest einer sekundären $\alpha$-Aminosäure mit höchstens 8 Kohlenstoffatomen ist, und

a, b, c, f und g unabhängig für 0 oder 1 stehen, sowie von nicht-toxischen Salzen und pharmakologisch anwendbaren Komplexen davon, dadurch gekennzeichnet, dass man ein Peptid der Formel

$(A_o)_a$-$(B)_b$-$(C)_c$-D-Phe-trp-Lys-Thr$(Y_o)$-E-
$[Thr(Y_o)]_f$-$[G(Y_o)]_g$ (II)

worin $A_o$ einen dem oben definierten Rest A entsprechenden Rest bedeutet, in welchem die $\alpha$-Aminogruppe des N-terminalen Aminosäurerestes eine $\alpha$-Aminoschutzgruppe X' tragen kann, $Y_o$ Wasserstoff oder eine Hydroxylschutzgruppe Y ist, und worin die übrigen Symbole die oben angegebenen Bedeutungen haben, mit einer Alkancarbonsäure $Ac_oOH$, worin $Ac_o$ einen dem oben definierten Acylrest Ac entsprechenden Rest bedeutet, in welchem vorhandene Amino- und Hydroxylgruppen mit Schutzgruppen X, X', bzw. Y versehen sein können, oder mit einem reaktionsfähigen Derivat einer solchen Säure behandelt und, wenn erwünscht oder notwendig ist, im erhaltenen Produkt durch Abspaltung der Schutzgruppen X, X' und Y Aminogruppen bzw. Hydroxylgruppen freisetzt.

3. Verfahren gemäss Anspruch 1 zur Herstellung von Acylpeptiden der allgemeinen Formel

$(A)_a$-$(B)_b$-$(C)_c$-D-Phe-trp-Lys(Ac)-Thr-E- $(Thr)_f$-$(G)_g$

worin Ac, A, B, C, D, E, G, trp, a, b, c, f und g die im Anspruch 2 angegebene Bedeutung haben, dadurch gekennzeichnet, dass man ein lineares Peptid der Formel

H-$[I_a]$-V

worin $I_a$ einen der im Anspruch 2 definierten Formel I entsprechenden Rest darstellt, in welchem die amidische Bindung zwischen zwei beliebigen benachbarten Aminosäureresten eines Peptidringes unterbrochen ist, und V für eine freie Hydroxylgruppe, eine durch eine Aktivierungsgruppe abgewandelte Hydroxylgruppe oder die Hydrazinogruppe -NH-$NH_2$ steht, cyclisiert, wobei gegebenenfalls vorhandene, an der Cyclisierungsreaktion nicht beteiligte Amino-, Carboxyl- und Hydroxylgruppen nach Bedarf in geschützter Form vorliegen und anschliessend freigesetzt werden.

4. Verfahren gemäss Anspruch 1 zur Herstellung von Acylpeptiden der allgemeinen Formel

$(A)_a$-$(B)_b$-$(C)_c$-D-Phe-trp-Lys(Ac)-Thr-E- $(Thr)_f$-$(G)_g$

worin Ac, A, B, C, D, E, G, trp, a, b, c, f und g die im Anspruch 2 angegebene Bedeutung haben, dadurch gekennzeichnet, das man ein entsprechendes lineares Peptid der Formel

$T_o$-$[I_s]$-$T_o$ (IV)

worin $I_s$ einen der im Anspruch 2 definierten Formel I entsprechenden Rest darstellt, in welchem die disulfidische Bindung zwischen den schwefelhaltigen Aminosäureresten unterbrochen ist, und $T_o$ für Wasserstoff oder eine Mercaptoschutzgruppe T steht, zur Bildung der Disulfid-Brücke, gegebenenfalls unter vorangehender oder gleichzeitiger Abspaltung der Mercaptoschutzgruppen T oxidiert, und gegebenenfalls in geschützter Form vorhandene Amino-, Carboxyl- und/oder Hydroxylgruppen freisetzt.

5. Verfahren gemäss einem der Ansprüche 1-4, dadurch gekennzeichnet, dass man ein Acylpeptid der allgemeinen Formel

A'-Cys-B-Asn-Phe-Phe-trp-Lys(Ac)-Thr-Phe-Thr-

Ser-cys-OH (IA)

worin A' für H-Ala-Gly-, Ac-Ala-Gly-, H- oder Acsteht und Ac, B, trp und cys die im Anspruch 2 angegebenen Bedeutungen haben, oder ein nicht-toxisches Salz oder einen pharmakologisch anwendbaren Komplex davon herstellt.

6. Verfahren gemäss einem der Ansprüche 1-3, dadurch gekennzeichnet, dass man ein Acylpeptid der allgemeinen Formel

Asn-Phe-Phe-$[trp_o]$-Lys(Ac)-Thr-Phe-NH-

$CH_2$-CH(U)-$CH_2$-CO IF

worin $trp_o$ für D-Trp oder D-(5F)Trp steht, Ac die im Anspruch 2 definierten Bedeutungen hat und U Wasserstoff oder einen gegebenenfalls substituierten cyclischen Hydrocarbylrest Ar bedeutet, oder ein nicht-toxisches Salz oder einen pharmakologisch anwendbaren Komplex davon herstellt.

7. Verfahren gemäss einem der Ansprüche 1-3, dadurch gekennzeichnet, dass man ein Acylpeptid der Formel

Asn-Phe-Phe-(D-Trp)-Lys(Ac)-Thr- Phe-Gaba

(IG)

worin Ac für den Rest einer in der Natur vorkommenden α-Aminosäure oder ihres D-Epimeren steht, oder ein nicht-toxisches Salz oder einen pharmakologisch anwendbaren Komplex davon herstellt.

8. Verfahren gemäss einem der Ansprüche 1-3, dadurch gekennzeichnet, dass man ein Acylpeptid der Formel IG, worin Ac für Glycyl, Leucyl oder Phenylalanyl steht, oder ein nicht-toxisches Salz oder einen pharmakologisch anwendbaren Komplex davon herstellt.

9. Verfahren gemäss einem der Ansprüche 1-3, dadurch gekennzeichnet, dass man ein Acylpeptid der Formel IG, worin Ac für Prolyl steht, oder ein nicht-toxisches Salz oder einen pharmakologisch anwendbaren Komplex davon herstellt.

10. Verfahren gemäss einem der Ansprüche 1-3, dadurch gekennzeichnet, dass man ein Acylpeptid der Formel IG, worin Ac für Lysyl oder $N^{\alpha}$-tert.-Butoxycarbonyllysyl steht, oder ein nicht-toxisches Salz oder einen pharmakologisch anwendbaren Komplex davon herstellt.

11. Verfahren zur Herstellung von pharmazeutischen Präparaten, enthaltend mindestens einen gemäss einem der Ansprüche 1-8 hergestellten Endstoff, auf nicht-chemischem Wege.

12. Verfahren zur Herstellung von pharmazeutischen Präparaten, enthaltend mindestens einen gemäss Anspruch 9 oder 10 hergestellten Endstoff, auf nicht-chemischem Wege.

**Claims for the contracting states:**
BE, CH, DE, FR, GB, IT, LI, LU, NL, SE

1. An acylpeptide derived from somatostatin or from a derivative thereof having analogous action in which the amino acids sequence is modified by omitting individual amino acids and/or exchanging them for other amino acids, and in which the ε-amino group of the lysine residue in the 9-position, and optionally also the ε-amino group of the lysine residue in the 4-position and/or the N-terminal α-amino group carries the acyl radical of an optionally substituted alkanecarboxylic acid, and salts and complexes thereof.

2. An acylpeptide according to claim 1 of the general formula

$(A)_a$-$(B)_b$-$(C)_c$-D-Phe-trp-Lys(Ac)-Thr-E- $(Thr)_f$-$(G)_g$
3 4 5 6 7 8 9 10 11 12 13

(I)

in which

Ac represents an acyl radical of an optionally substituted alkanecarboxylic acid, present at the free amino group,

A represents the radical of the partial formula

H-$Ala^1$-$Gly^2$-$Cys^3$- -$cys^{14}$-OH, Ac-$Ala^1$-$Gly^2$-$Cys^3$- -$cys^{14}$-OH, H-$Cys^3$- -$cys^{14}$-OH, Ac-$Cys^3$- -$cys^{14}$-OH oder Bmp- -$cys^{14}$-OH

(wherein cys represents L-Cys or D-Cys and Bmp represents the desaminocysteine residue), or the residue of an $\omega$-amino-lower alkanecarboxylic acid of the partial formula -NH-CH(R)-$(CH_2)_n$-CO- (wherein n represents 0 or an integer from 1 to 6 and R represents hydrogen or carboxyl) which, if $n = 2$ and R is hydrogen, can also be substituted by a cyclic hydrocarbyl radical and, in that case, is designated by the symbol Gaba(Ar),

B represents Lys, Lys(Ac) or Lys(X) (wherein X is an $\epsilon$-amino-protecting group),

C represents Asn, Ala or His,

D represents Phe or, if no sulphur-containing amino acid residues are present in the radical A, together with E may represent the radical

-$Cys^{6}$- $Cys^{11}$-

trp represents L-Trp, D-Trp or an analogous radical, which carries in the indole nucleus a halogen atom,

E represents Phe or Tyr or, together with D, has the meaning given above,

G represents L-Ser, D-Ser or the residue of a secondary $\alpha$-amino acid having a maximum of 8 carbon atoms, and

a, b, c, f and g each represents, independently of one another, 0 or 1, and non-toxic salts and pharmacologically acceptable complexes thereof.

3. An acylpeptide according to claim 2 of the general formula

A'-Cys-B-Asn-Phe-Phe-trp-Lys(Ac)-Thr-Phe-Thr-

Ser-cys-OH (IA)

in which A' represents H-Ala-Gly-, Ac-Ala-Gly-, H- or Ac-, and Ac, B, trp and cys have the meanings given in claim 2.

4. An acylpeptide according to claim 2 of the general formula

Asn-Phe-Phe-[$trp_o$]-Lys(Ac)-Thr-Phe-NH-

$CH_2$-CH(U)-$CH_2$-CO IF

in which $trp_o$ represents D-Trp or D-(5F)Trp, Ac has the meanings defined in claim 2 and U represents hydrogen or an optionally substituted cyclic hydrocarbyl radical Ar.

5. An acylpeptide according to claim 4, in which U represents hydrogen and Ac represents the residue of a naturally occurring $\alpha$-amino acid or of a closely related analogue thereof and trp has the meanings defined in claim 4.

6. An acylpeptide according to claim 4 of the formula IF, in which $trp_o$ represents D-Trp, U represents hydrogen and Ac represents glycyl, leucyl or phenylalanyl.

7. An acylpeptide according to claim 4 of the formula IF, in which $trp_o$ represents D-Trp, U represents hydrogen and Ac represents prolyl.

8. An acylpeptide according to claim 4 of the formula IF, in which $trp_o$ represents D-Trp, U represents hydrogen and Ac represents $N^{\alpha}$-tert.-butoxycarbonyllysyl or lysyl.

9. Process for the manufacture of acylpeptides derived from somatostatin or from a derivative thereof having analogous action in which the amino acids sequence is modified by omitting individual amino acids and/or by exchanging them for other amino acids, and in which the $\epsilon$-amino group of the lysine residue in the 9-position, and optionally also the $\epsilon$-amino group of the lysine residue in the 4-position and/or the N-terminal $\alpha$-amino group carries the acyl radical of an optionally substituted alkanecarboxylic acid, and salts and complexes thereof, characterised in that a corresponding peptide having a free $\epsilon$-amino group in the lysine residue in the 9-position is acylated, optionally while temporarily protecting any free hydroxy groups or other free amino groups, or a linear peptide corresponding to the acylpeptide is cyclised, optionally while temporarily protecting any free hydroxyl, carboxyl and/or amino groups, and, if desired, compounds obtained in the form of salts are converted into the corresponding free form or a resulting free compound is converted into a corresponding salt, and/or, if desired, a resulting compound is converted into a complex thereof.

10. A pharmaceutical preparation containing an acylpeptide defined in any one of claims 1 to 6.

11. A pharmaceutical preparation containing an acylpeptide defined in claim 7 or 8.

12. An acylpeptide defined in any one of claims 1 to 6 as an antidiabetic.

13. An acylpeptide defined in claim 7 or 8 as an antidiabetic.

**Claims for the contracting state:**
AT

1. Process for the manufacture of acylpeptides derived from somatostatin or from a derivative thereof having analogous action in which the amino acids sequence is modified by omitting individual amino acids and/or by exchanging them for other amino acids, and in which the $\epsilon$-amino group of the lysine residue in the 9-position, and optionally also the $\epsilon$-amino group of the lysine residue in the 4-position and/or the N-terminal $\alpha$-amino group carries the acyl radical of an optionally substituted alkanecarboxylic acid, and salts and complexes thereof, characterised in that a corresponding peptide having a free $\epsilon$-amino group in the lysine residue in the 9-position is acylated, optionally while temporarily protecting any free hydroxy groups or other free amino groups, or a linear peptide corresponding to the acylpeptide is cyclised, optionally while temporarily protecting any free hydroxyl, carboxyl and/or amino groups, and, if desired, compounds obtained in the form of salts are converted into the corresponding free form or a resulting free compound is converted into a corresponding salt, and/or, if desired, a resulting compound is converted into a complex thereof.

2. Process according to claim 1 for the manufacture of acylpeptides of the general formula

$(A)_a$-$(B)_b$-$(C)_c$-D-Phe-trp-Lys(Ac)-Thr-E- $(Thr)_f$-$(G)_g$
3 4 5 6 7 8 9 10 11 12 13
(I)

in which

Ac represents an acyl radical of an optionally substituted alkanecarboxylic acid, present at the free amino group,

A represents the radical of the partial formula

H-Ala$^1$-Gly$^2$-Cys$^3$- -cys$^{14}$-OH, Ac-Ala$^1$-Gly$^2$-Cys$^3$- -cys$^{14}$-OH, H-Cys$^3$- -cys$^{14}$-OH, Ac-Cys$^3$- -cys$^{14}$-OH or Bmp- -cys$^{14}$-OH

(wherein cys represents L-Cys or D-Cys and Bmp represents the desaminocysteine residue), or the residue of an $\omega$-amino-lower alkanecarboxylic acid of the partial formula $-NH-CH(R)-(CH_2)_n-CO-$ (wherein n represents 0 or an integer from 1 to 6 and R represents hydrogen or carboxyl) which, if $n = 2$ and R is hydrogen, can also be substituted by a cyclic hydrocarbyl radical and, in that case, is designated by the symbol Gaba(Ar),

B represents Lys, Lys(Ac) or Lys(X) (wherein X is an $\epsilon$-amino-protecting group),

C represents Asn, Ala or His,

D represents Phe or, if no sulphur-containing amino acid residues are present in the radical A, together with E may represent the radical

-Cys$^6$- Cys$^{11}$-,

trp represents L-Trp, D-Trp or an analogous radical, which carries in the indole nucleus, for example in the 5-position, a halogen atom, especially fluorine,

E represents Phe or Tyr or, together with D, has the meaning given above,

G represents L-Ser, D-Ser or the residue of a secondary $\alpha$-amino acid having a maximum of 8 carbon atoms, and

a, b, c, f and g each represents, independently of one another, 0 or 1, and non-toxic salts and pharmacologically acceptable complexes thereof, characterised in that a peptide of the formula

(A$_o$)$_a$-(B)$_b$-(C)$_c$-D-Phe-trp-Lys-Thr(Y$_o$)-E-[Thr(Y$_o$)]$_f$-[G(Y$_o$)]$_g$ (II)

in which A$_o$ represents a radical corresponding to the radical A defined above in which the $\alpha$-amino group of the N-terminal amino acid residue may carry an $\alpha$-amino-protecting group X', Y$_o$ represents hydrogen or a hydroxyl-protecting group Y, and in which the other symbols have the meanings given above, is treated with an alkanecarboxylic acid Ac$_o$OH, in which Ac$_o$ represents a radical corresponding to the acyl radical Ac defined above in which any amino and hydroxyl groups present may be provided with protecting groups X and X', and Y respectively, or with a reactive derivative of such an acid, and, if desired or necessary, in the resulting product the amino groups and hydroxyl groups are liberated by splitting off the protecting groups X and X', and Y respectively.

3. Process according to claim 1 for the manufacture of acylpeptides of the general formula

(A)$_a$-(B)$_b$-(C)$_c$-D-Phe-trp-Lys(Ac)-Thr-E- (Thr)$_f$-(G)$_g$

in which Ac, A, B, C, D, E, G, trp, a, b, c, f and g have the meanings given in claim 2, characterised in that a linear peptide of the formula

H-[I$_a$]-V

in which I$_a$ represents a radical corresponding to the formula I defined in claim 2 in which the amide bond between any two adjacent amino acid residues of a peptide ring is interrupted, and V represents a free hydroxyl group, a hydroxyl group modified by an activating group or represents the hydrazino group $-NH-NH_2$, is cyclised, any amino, carboxyl and hydroxyl groups present that do not participate in the cyclisation reaction being, as required, in protected form and liberated subsequently.

4. Process according to claim 1 for the manufacture of acylpeptides of the general formula

(A)$_a$-(B)$_b$-(C)$_c$-D-Phe-trp-Lys(Ac)-Thr-E- (Thr)$_f$-(G)$_g$

in which Ac, A, B, C, D, E, G, trp, a, b, c, f and g have the meanings given in claim 2, characterised in that a corresponding linear peptide of the formula

T$_o$-[I$_s$]-T$_o$ (IV)

in which I$_s$ represents a radical corresponding to the formula I defined in claim 2 in which the disulphide bond between the sulpher-containing amino acid residues is interrupted, and T$_o$ represents hydrogen or a mercapto-protecting group T, is oxidised to form the disulphide bridge, optionally having split off the mercapto-protecting groups T beforehand or splitting them off at the same time, and any amino, carboxyl and/or hydroxyl groups present in protected form are liberated.

5. Process according to any one of claims 1 to 4, characterised in that an acylpeptide of the general formula

A'-Cys-B-Asn-Phe-Phe-trp-Lys(Ac)-Thr-Phe-Thr-Ser-cys-OH (IA)

in which A' represents H-Ala-Gly-, Ac-Ala-Gly-, H- or Ac-, and Ac, B, trp and cys have the meanings given in claim 2, or a non-toxic salt or a pharmacologically acceptable complex thereof is manufactured.

6. Process according to any one of claims 1 to 3, characterised in that an acylpeptide of the general formula

Asn-Phe-Phe-[trp$_o$]-Lys(Ac)-Thr-Phe-NH-$CH_2$-CH(U)-$CH_2$-CO IF

in which trp$_o$ represents D-Trp or D-(5F)Trp, Ac has the meanings defined in claim 2 and U represents hydrogen or an optionally substituted cyclic hydrocarbyl radical Ar, or a non-toxic salt or a pharmacologically acceptable complex thereof is manufactured.

7. Process according to any one of claims 1 to 3, characterised in that an acylpeptide of the formula

Asn-Phe-Phe-(D-Trp)-Lys(Ac)-Thr- Phe-Gaba (IG)

in which Ac represents the residue of a naturally occurring α-amino acid or of the D-epimer thereof, or a non-toxic salt or a pharmacologically acceptable complex thereof is manufactured.

8. Process according to any one of claims 1 to 3, characterised in that an acylpeptide of the formula IG in which Ac represents glycyl, leucyl or phenylalanyl, or a non-toxic salt or a pharmacologically acceptable complex thereof is manufactured.

9. Process according to any one of claims 1 to 3, characterised in that an acylpeptide of the formula IG in which Ac represents prolyl, or a non-toxic salt or a pharmacologically acceptable complex thereof is manufactured.

10. Process according to any one of claims 1 to 3, characterised in that an acylpeptide of the formula IG in which Ac represents lysyl or $N^{\alpha}$-tert.-butoxy-carbonyllysyl, or a non-toxic salt or a pharmacologically acceptable complex thereof is manufactured.

11. Process for the manufacture of pharmaceutical preparations containing at least one end product manufactured according to any one of claims 1-8 by non-chemical means.

12. Process for the manufacture of pharmaceutical preparations containing at least one end product manufactured according to claim 9 or 10 by non-chemical means.

**Revendications pour les Etats contractants:**
BE, CH, DE, FR, GB, IT, LI, LU, NL, SE

1. Acylpeptide, dérivant de la somatostatine ou d'un dérivé de la somatostatine à activité analogue dont la séquence d'aminoacides a été modifiée par la suppression d'aminoacides individuels et/ou leur échange contre d'autres aminoacides, et qui se caractérise en ce que le groupe epsilon-amino du reste de lysine en position 9, et éventuellement également le groupe epsilon-amino du reste de lysine en position 4 et/ou le groupe alpha-amino sur l'azote terminal, porte le reste acyle d'un acide alcane-carboxylique éventuellement substitué, ses sels et complexes.

2. Acylpeptide selon la revendication 1 de formule générale:

$(A)_a$-$(B)_b$-$(C)_c$-D-Phe-trp-Lys(Ac)-Thr-E- $(Thr)_f$-$(G)_g$
3 4 5 6 7 8 9 10 11 12 13

dans laquelle: (I)

Ac représente un reste acyle d'un acide alcane-carboxylique éventuellement substitué qui se trouve sur le groupe amino libre,

A représente le reste de formule partielle:

H-Ala[1]-Gly[2]-$Cys^3$- -$cys^{14}$-OH, Ac-Ala[1]-Gly[2]-$Cys^3$- -$cys^{14}$-OH, H-$Cys^3$- -$cys^{14}$-OH, Ac-$Cys^3$- -$cys^{14}$-OH ou Bmp- -$cys^{14}$-OH

(dans laquelle cys représente L-Cys ou D-Cys et Bmp représente le reste de la désaminocystéine), ou le reste d'un acide oméga-amino-(alcane inférieur) carboxylique de formule partielle -NH-CH(R)-$(CH_2)_n$-CO- (dans laquelle n est un nombre entier de 0 à 6, et R représente l'hydrogène ou un groupe carboxyle), qui peut également, lorsque n = 2 et R représente l'hydrogène, être substitué par un reste hydrocarboné cyclique et est représenté dans un tel cas par le symbole Gaba(Ar),

B représente Lys(Ac) ou Lys(X) (X étant un groupe protecteur du groupe epsilon-amino),

C représente Asn, Ala ou His,

D représente Phe ou bien, lorsqu'il n'y a pas de restes d'aminoacides soufrés dans le reste A, il peut former avec E le reste

-$Cys^6$- $Cys^{11}$-,

trp représente L-Trp, D-Trp ou un reste analogue portant un halogène dans le noyau indole,

E représente Phe ou Tyr ou forme avec D le reste indiqué ci-dessus,

G représente L-Ser, D-Ser ou le reste d'un alpha-aminoacide secondaire contenant au maximum 8 atomes de carbone, et

a, b, c, f et g représentent chacun, indépendamment, 0 ou 1, ainsi que les sels non toxiques et complexes de ces composés acceptables pour l'usage pharmaceutique.

3. Acylpeptide selon la revendication 2 de formule générale:

A'-Cys-B-Asn-Phe-Phe-trp-Lys(Ac)-Thr-Phe-Thr-Ser-cys-OH (IA)

dans laquelle:

A' représente H-Ala-Gly-, Ac-Ala-Gly-, H- ou Ac-, et Ac, B, trp ou cys ont les significations indiquées dans la revendication 2.

4. Acylpeptide selon la revendication 2 de formule générale:

Asn-Phe-Phe-$[trp_o]$-Lys(Ac)-Thr-Phe-NH-$CH_2$-CH(U)-$CH_2$-CO. IF

dans laquelle:

$trp_o$ représente D-Trp ou D-(5F)Trp,

Ac a les significations indiquées dans la revendication 2, et

U représente l'hydrogène ou un reste hydrocarboné cyclique éventuellement substitué par Ar.

5. Acylpeptide selon la revendication 4, dans lequel U représente l'hydrogène et Ac le reste d'un alpha-aminoacide existant dans la nature ou d'un composé analogue étroitement apparenté, et trp a la signification indiquée dans la revendication 4.

6. Acylpeptide selon la revendication 4, de formule IF dans laquelle $trp_o$ représente D-Trp, U représente l'hydrogène et Ac représente un reste glycyle, leucyle ou phénylalanyle.

7. Acylpeptide selon la revendication 4, de formule IF dans laquelle $trp_o$ représente D-Trp, U représente l'hydrogène et Ac le reste prolyle.

8. Acylpeptide selon la revendication 4, de formule IF dans laquelle $trp_o$ représente D-Trp, U représente l'hydrogène et Ac représente un reste $N^{alpha}$-tert.-butoxycarbonyl-lysyle ou lysyle.

9. Procédé de préparation d'acylpeptides dérivant de la somatostatine ou d'un dérivé de ce composé à activité analogue dont la séquence d'aminoacides a été modifiée par suppression d'aminoacides individuels et/ou par échange de ces aminoacides contre d'autres aminoacides, et dans lesquels le groupe epsilon-amino du reste de lysine en position 9 et éventuellement également le groupe epsilon-amino du reste de lysine en position 4 et/ou le groupe alpha-amino à azote terminal porte le reste acyle d'un acide alcane-carboxylique éventuellement substitué, ainsi que des sels et complexes de ces composés, caractérisé en ce que l'on acyle un peptide correspondant dont le groupe epsilon-amino du reste de lysine en position 9 est libre, éventuellement avec protection transitoire des groupes hydroxy libres présents ou des autres groupes amino libres, ou bien on cyclise un peptide linéaire correspondant à l'acylpeptide, éventuellement avec protection transitoire des groupes hydroxy, carboxyles et/ou amino libres présents et, si on le désire, on convertit un composé obtenu en son complexe.

10. Composition pharmaceutique contenant un acylpeptide défini dans l'une quelconque des revendications 1 à 6.

11. Composition pharmaceutique contenant un acylpeptide défini dans la revendication 7 ou 8.

12. Acylpeptide défini dans l'une quelconque des revendications 1 à 6, en tant qu'antidiabétique.

13. Acylpeptide défini dans la revendication 7 ou 8, en tant qu'antidiabétique.

**Revendications pour l'Etat contractant:**
AT

1. Procédé de préparation d'acylpeptides dérivant de la somatostatine ou d'un dérivé de ce composé à activité analogue dont la séquence d'aminoacides a été modifiée par suppression d'aminoacides individuels et/ou échange de ces aminoacides contre d'autres aminoacides, et ont pour caractéristique de porter sur le groupe epsilon-amino du reste de lysine en position 9, et éventuellement également sur le groupe epsilon-amino du reste de lysine en position 4 et/ou sur le groupe alpha-amino à azote terminal, le reste acyle d'un acide alcane-carboxylique éventuellement substitué, ainsi que des sels et complexes de ces composés, caractérisé en ce que l'on acyle un peptide correspondant dont le groupe epsilon-amino du reste de lysine en position 9 est libre, éventuellement avec protection transitoire des groupes hydroxy libres présents ou des autres groupes amino libres, ou bien on cyclise un peptide linéaire correspondant à l'acylpeptide, éventuellement avec protection transitoire des groupes hydroxy, carboxyles et/ou amino libres présents et, si on le désire, on convertit les composés obtenus à l'état de sels en la forme libre correspondante ou un composé libre obtenu en un sel correspondant, et/ou si on le désire, on convertit un composé obtenu en son complexe.

2. Procédé selon la revendication 1, pour la préparation des acylpeptides de formule générale:

$(A)_a$-$(B)_b$-$(C)_c$-D-Phe-trp-Lys(Ac)-Thr-E-$(Thr)_f$-$(G)_g$
3 4 5 6 7 8 9 10 11 12 13

(I)

dans laquelle:

Ac représente le reste acyle d'un acide alcane-carboxylique éventuellement substitué qui se trouve sur le groupe amino libre,

A représente le reste de formule partielle:

H-$Ala^1$-$Gly^2$-$Cys^3$- -$cys^{14}$-OH, Ac-$Ala^1$-$Gly^2$-$Cys^3$- -$cys^{14}$-OH, H-$Cys^3$- -$cys^{14}$-OH,

Ac-$Cys^3$- -$cys^{14}$-OH ou Bmp- -$cys^{14}$-OH

(dans laquelle cys représente L-Cys ou D-Cys et Bmp représente le reste de la désaminocystéine) ou le reste d'un acide oméga-amino-(alcane inférieur)-carboxylique de formule partielle $-NH-CH(R)-(CH_2)_n-CO-$ (dans laquelle n est un nombre entier de 0 à 6 et R représente l'hydrogène ou un groupe carboxyle), qui peut également, lorsque n = 2 et R représente l'hydrogène, être substitué par un reste hydrocarboné cyclique et est désigné dans un tel cas par le symbole Gaba(Ar),

B représente Lys, Lys(Ac) ou Lys(X) (X étant un groupe protecteur du groupe epsilon-amino),

C représente Asn, Ala ou His,

D représente Phe ou bien, lorsqu'il n'y a pas de restes d'aminoacides soufrés dans le reste A, il peut former avec E le reste

-$Cys^6$- $Cys^{11}$-,

trp représente L-Trp, D-Trp ou un reste analogue qui porte dans le noyau indole, par exemple en position 5, un halogène, en particulier le fluor,

E représente Phe ou Tyr ou forme avec D le groupe indiqué ci-dessus,

G représente L-Ser, D-Ser ou le reste d'un alpha-aminoacide secondaire contenant au maximum 8 atomes de carbone, et

a, b, c, f et g représentent indépendamment 0 ou 1, et de leurs sels non toxiques et complexes acceptables pour l'usage pharmaceutique, caractérisé en ce que l'on traite un peptide de formule:

$(A_o)_a$-$(B)_b$-$(C)_c$-D-Phe-trp-Lys-Thr$(Y_o)$-E-
$[Thr(Y_o)]_f$-$[G(Y_o)]_g$ (II)

dans laquelle $A_o$ représente un reste correspondant au reste A défini ci-dessus, dans lequel le groupe alpha-amino du reste d'aminoacide à azote terminal peut porter un groupe protecteur X' du groupe alpha-amino, $Y_o$ représente l'hydrogène ou un groupe protecteur Y du groupe hydroxy, et les autres symboles ont les significations indiquées ci-dessus, avec un acide alcane-carboxylique $Ac_oOH$ dans lequel $Ac_o$ est un reste correspondant au reste acyle Ac défini ci-dessus, dans lequel les groupes amino et hydroxy

présents peuvent porter des groupes protecteurs respectifs X, X' et Y, ou avec un dérivé réactif d'un tel acide et, si on le désire ou si c'est nécessaire, on libère les groupes amino et les groupes hydroxy du produit obtenu par élimination des groupes protecteurs respectifs X, X' et Y.

3. Procédé selon la revendication 1 pour préparer les acylpeptides de formule générale:

$(A)_a$-$(B)_b$-$(C)_c$-D-Phe-trp-Lys(Ac)-Thr-E- $(Thr)_f$-$(G)_g$

dans laquelle Ac, A, B, C, D, E, G, trp, a, b, c, f et g ont les significations indiquées dans la revendication 2, caractérisé en ce que l'on cyclise un peptide linéaire de formule:

H-$[I_a]$-V

dans laquelle $I_a$ est un reste répondant à la formule I définie dans la revendication 2 et dans lequel la liaison amide entre deux restes d'aminoacides quelconques voisins d'un cycle peptidique est interrompue, et V représente un groupe hydroxy libre, un groupe hydroxy modifié par un groupe activant ou le groupe hydrazino $-NH-NH_2$, les groupes amino, carboxyles et hydroxy éventuellement présents et ne participant pas à la réaction de cyclisation étant à l'état protégé si c'est nécessaire et ensuite libérés.

4. Procédé selon la revendication 1, pour préparer les acylpeptides de formule générale:

$(A)_a$-$(B)_b$-$(C)_c$-D-Phe-trp-Lys(Ac)-Thr-E- $(Thr)_f$-$(G)_g$

dans laquelle Ac, A, B, C, D, E, G, trp, a, b, c, f et g ont les significations indiquées dans la revendication 2, caractérisé en ce que l'on oxyde un peptide linéaire correspondant de formule:

$T_o$-$[I_s]$-$T_o$ (IV)

dans laquelle $I_s$ représente un reste répondant à la formule I définie dans la revendication 2 dans lequel la liaison disulfure entre les restes d'aminoacides soufrés est interrompue, et $T_o$ représente l'hydrogène ou un groupe protecteur T du groupe mercapto, pour formation du pont disulfure, éventuellement avec séparation préalable ou simultanée du groupe protecteur T du groupe mercapto, et on libère les groupes amino, carboxyles et/ou hydroxyles éventuellement à l'état protégé.

5. Procédé selon l'une quelconque des revendications 1 à 4, caractérisé en ce que l'on prépare un acylpeptide de formule générale:

A'-Cys-B-Asn-Phe-Phe-trp-Lys(Ac)-Thr-Phe-Thr-Ser-cys-OH (IA)

dans laquelle A' représente H-Ala-Gly, Ac-Ala-Gly, H- ou Ac-, et Ac, B, trp et cys ont les significations indiquées dans la revendication 2, ou un sel non toxique ou un complexe acceptable pour l'usage pharmaceutique d'un tel composé.

6. Procédé selon l'une quelconque des revendications 1 à 3, caractérisé en ce que l'on prépare un acylpeptide de formule générale:

Asn-Phe-Phe-$[trp_o]$-Lys(Ac)-Thr-Phe-NH-$CH_2$-CH(U)-$CH_2$-CO IF

dans laquelle $trp_o$ représente D-Trp ou D-(5F)Trp, Ac a les significations indiquées dans la revendication 2 et U représente l'hydrogène ou un reste hydrocarboné cyclique éventuellement substitué par Ar, ou un sel non toxique ou un complexe acceptable pour l'usage pharmaceutique d'un tel composé.

7. Procédé selon l'une quelconque des revendications 1 à 3, caractérisé en ce que l'on prépare un acylpeptide de formule:

Asn-Phe-Phe-(D-Trp)-Lys(Ac)-Thr- Phe-Gaba (IG)

dans laquelle Ac représente le reste d'un alpha-aminoacide existant à l'état naturel ou de son D-épimère, ou un sel non toxique ou un complexe acceptable pour l'usage pharmaceutique d'un tel composé.

8. Procédé selon l'une quelconque des revendications 1 à 3, caractérisé en ce que l'on prépare un acylpeptide de formule IG dans laquelle Ac représente un reste glycyle, leucyle ou phénylalanyle, ou un sel non toxique ou un complexe acceptable pour l'usage pharmaceutique d'un tel composé.

9. Procédé selon l'une quelconque des revendications 1 à 3, caractérisé en ce que l'on prépare un acylpeptide de formule IG dans laquelle Ac représente le reste prolyle, ou un sel non toxique ou un complexe acceptable pour l'usage pharmaceutique d'un tel composé.

10. Procédé selon l'une quelconque des revendications 1 à 3, caractérisé en ce que l'on prépare un acylpeptide de formule IG dans laquelle Ac représente le groupe lysyle ou le groupe $N^{alpha}$-tert.-butoxycarbonyllysyle, ou un sel non toxique ou un complexe acceptable pour l'usage pharmaceutique d'un tel composé.

11. Procédé de préparation de compositions pharmaceutiques contenant au moins un produit final préparé selon l'une quelconque des revendications 1 à 8, par voie non chimique.

12. Procédé de préparation de compositions pharmaceutiques contenant au moins un produit final préparé selon la revendication 9 ou 10, par voie non chimique.